# EUROPEAN PATENT SPECIFICATION

(11) **EP 4 281 072 B1**
(45) Date of publication and mention of the grant of the patent: **09.12.2026**
(21) Application number: 22702187.0
(22) Date of filing: 19.01.2022
(51) Int. Cl.: A61K 31/4439, A61K 45/06, A61P 35/00, A61P 35/02

(54) **FERROPORTIN-INHIBITORS FOR THE USE IN THE TREATMENT OF MYELODYSPLASTIC SYNDROMES (MDS)**
FERROPORTININHIBITOREN ZUR VERWENDUNG BEI DER BEHANDLUNG VON MYELODYSPLASTISCHEN SYNDROMEN (MDS)
INHIBITEURS DE FERROPORTINE UTILISÉS POUR LE TRAITEMENT DES SYNDROMES MYÉLODYSPLASIQUES (MDS)

(30) Priority: 20.01.2021 US 202163139586 P; 28.04.2021 EP 21170897
(43) Date of publication of application: 29.11.2023
(73) Proprietor: VIFOR (INTERNATIONAL) AG, 9001 St. Gallen (CH)
(72) Inventor: VINCHI, Francesca, New York, New York 10065 (US); MANOLOVA, Vania, 9001 St. Gallen (CH); DÜRRENBERGER, Franz, 9001 St. Gallen (CH)
(74) Representative: Gille Hrabal Partnerschaftsgesellschaft mbB Patentanwälte
(86) International application number: PCT/EP2022/051108
(87) International publication number: WO 2022/157185

(56) References cited:
- WO-A1-2017/068090
- WO-A1-2018/192973
- MANOLOVA VANIA ET AL: "Oral ferroportin inhibitor ameliorates ineffective erythropoiesis in a model of [beta]-thalassemia", vol. 130, no. 1, 2 January 2020 (2020-01-02), GB, pages 491 - 506, XP055844753, ISSN: 0021-9738, Retrieved from the Internet <URL:https://www.ncbi.nlm.nih.gov/pmc/articles/PMC6934209/pdf/jci-130-129382.pdf> DOI: 10.1172/JCI129382
- FRANK RICHARD ET AL: "Oral ferroportin inhibitor VIT-2763: First-in-human, phase 1 study in healthy volunteers", AMERICAN JOURNAL OF HEMATOLOGY, vol. 95, no. 1, 19 November 2019 (2019-11-19), US, pages 68 - 77, XP055657378, ISSN: 0361-8609, DOI: 10.1002/ajh.25670

## Description

### INTRODUCTION

The invention relates to a compound which acts as ferroportin inhibitor, for treating myelodysplastic syndromes (MDS) and the symptoms and pathological conditions associated therewith.

### BACKGROUND AND PRIOR ART

Iron is an essential element for almost all organisms and its relevance lies in its key role in erythropoiesis and oxygen transport. The balance of the iron metabolism is primarily regulated on the level of iron recovery from haemoglobin of ageing erythrocytes, from iron stores in the liver and the duodenal absorption of dietary iron. Elemental iron is taken up by duodenal enterocytes via specific transport systems (DMT-1, ferroportin), transferred into the blood circulation and thereby conveyed to the appropriate tissues and organs bound to its carrier transferrin. In the human body, iron is of great importance, *inter alia* for oxygen transport, oxygen uptake, cell functions such as mitochondrial electron transport, cognitive functions, etc. and ultimately for the entire energy metabolism. Mammalian organisms are unable to remove or excrete iron from the body through an active system. Iron homeostasis is controlled by the hepatic peptide hormone hepcidin, which regulates the activity of the only know iron exporter ferroportin and thus iron release from macrophages, hepatocytes and enterocytes. Hepcidin controls iron absorption via the intestine and placenta and iron recycling from the reticuloendothelial system. Hepcidin production is directly regulated by iron level, i.e. if the organism is supplied with sufficient or excess iron and oxygen, more hepcidin is produced; if iron and oxygen levels are low, or in case of increased erythropoiesis less hepcidin is produced. In the small intestinal mucosal cells and macrophages hepcidin binds to ferroportin, thus blocking its export function and promoting its internalization and degradation. Through this mechanism hepcidin reduces iron efflux from cells to the bloodstream. The transport protein ferroportin is a transmembrane protein consisting of 571 amino acids which is expressed in the liver, spleen, kidneys, heart, intestine and placenta. In particular, ferroportin is localized in the basolateral membrane of intestinal epithelial cells. Ferroportin thus acts to export dietary iron into the blood. If hepcidin binds to ferroportin, ferroportin is transported into the interior of the cell, where its breakdown takes place so that the release of iron from the cells is then blocked. If ferroportin is inactivated or inhibited, by hepcidin, so that it is unable to export the iron which is stored in the mucosal cells, the absorption of iron in the intestine is blocked. A decrease of hepcidin results in an increase of active ferroportin, thus allowing an enhanced dietary iron absorption and release of stored iron, and leading to increased serum iron level.

In pathological cases an increased iron level leads to iron overload. For example, excessive iron uptake in organs, such as liver and heart, leads to accumulation of iron. Further, iron accumulation in brain has been observed in patients suffering from neurodegenerative diseases such as for example Alzheimer's disease and Parkinson's disease. The major portion of circulating iron is associated with transferrin, a classical iron transporting molecule, which prevents the formation of free reactive iron. Iron fractions not bound to transferrin (or to the other traditional iron binding molecules like haem, apoferritin, hemosiderin etc.) are collectively referred to as non-transferrin bound iron (NTBI). In a further aspect of iron overload states and diseases many problems and pathological conditions arise from excess levels of free iron in the circulation, i.e. NTBI.

A key detrimental aspect of such excess of free iron is the undesired formation of radicals. In particular, iron (II) ions catalyze the formation (inter alia via Fenton reaction) of reactive oxygen species (ROS). ROS cause damage to DNA, lipids, proteins and carbohydrates which has far-reaching effects in cells, tissue and organs. The formation of ROS is well known and described in the literature to cause the so-called oxidative stress. NTBI is widely described to exhibit high propensity to induce ROS, having potential toxicity on cell and major organs, including heart, liver, pancreas, kidney and bone marrow. Accordingly, iron overload is known to cause tissue and organ damage, such as e.g. cardiac, liver and endocrine damage (Vinchi, Hell, Platzbecker "Controversies on the consequences of iron overload and chelation in MDS" Hemasphere, 27;4(3), 2020; Patel M. et al. "Non Transferrin Bound Iron: Nature, Manifestations and Analytical Approaches for Estimation" Ind. J. Clin. Biochem., 2012; 27(4): 322-332 *and* Brissot P. et al. Review "Non-transferrin bound iron: A key role in iron overload and iron toxicity" Biochimica et Biophysica Acta, 2012; 1820, 403-410*).*

Myelodysplastic syndromes (MDS) are a group of heterogeneous clonal bone marrow disorders, characterized by ineffective hematopoiesis leading to peripheral blood cytopenias, and a risk of leukemia transformation. MDS is one of the most frequently encountered acquired bone marrow failure syndromes in adults. Genetic and epigenetic changes that affect hematopoietic stem cells (HSCs) and alterations in the hematopoietic niche resulting in degeneration and apoptosis of hematopoietic stem and progenitor cells (HSPCs), mainly contribute to ineffective hematopoiesis.

MDS refer to a group of cancers in which HSPCs in the bone marrow do not mature, so do not become healthy blood cells. Typically, no symptoms are seen in early stages, but later symptoms may include feeling tired, shortness of breath, bleeding disorders, anemia, and frequent infections. Some types of MDS may develop into acute myeloid leukemia. In MDS the production of blood cells is inefficient, resulting in inadequate number of red blood cell, platelet, and white blood cells. Some MDS types are characterized by an increase in immature blood cells, called myeloblasts, in the bone marrow and/or blood. The types of MDS are based on specific changes in blood cells and bone marrow.

The International Prognostic Scoring System (IPSS) and the Revised IPSS (IPSS-R) characterize different classes of MDS. Lower-risk MDS are defined according to the IPSS as being of low or intermediate 1 risk, or according to the Revised IPSS [IPSS-R] as being of very low, low, or intermediate risk.

Lower-risk myelodysplastic syndromes according to the IPSS or IPSS-R most commonly manifest with symptomatic anemia. In particular in elderly persons, chronic anemia is associated with multiple complications, including cardiovascular complications, increased risks of falls and bone fracture, and shorter survival. A high proportion of patients with lower-risk myelodysplastic syndromes eventually become dependent on red-cell transfusions (transfusion-dependent), a situation that is associated with reduced quality of life and overall survival.

With the increasing feasibility of sequencing and identification of somatic gene mutations in clinical practice it was possible to identify further MDS subtypes defined by a genetic abnormality. Such subtypes are MDS with isolated del(5q) or MDS with SF3B1 mutation and are further described in a special report paper by L. Malcovati et al.: "SF3B1-mutant MDS as a distinct disease subtype: a proposal from the International Working Group for the Prognosis of MDS"; Blood, Vol. 136, No. 2, 157-170, 2020*.*

The common biological characteristic of low-risk MDS includes a defect in hematopoietic stem and progenitor cell self-renewal and differentiation, resulting in cytopenias. Approximately 60% to 80% of patients with MDS experience symptomatic anemia, and 80% to 90% of anemic MDS patients require red blood cell (RBC) transfusions as supportive therapy.

Iron overload is common in MDS, as consequence of increased intestinal iron absorption to support the expanded erythropoiesis and chronic RBC transfusions, which are often essential to correct the anemia in this patient population.

The main drivers of iron overload in MDS patients are ineffective erythropoiesis and blood transfusion therapy. Iron overload starts to develop in MDS patients before they become transfusion-dependent. That means, MDS patients might develop iron overload even before receiving transfusions, because of their underlying ineffective erythropoiesis, which triggers enhanced iron absorption to support the expanded erythron in an attempt to recover the anemia. The improvement of anemia through RBC transfusions is a central point of supportive care in MDS and typically, transfusions remain the dominant cause of iron overload in this patient population. Ineffective erythropoiesis leads to the suppression of the iron hormone hepcidin, which in turn triggers unrestrained iron absorption through duodenal enterocytes. This mechanism allows enhanced iron influx into the circulation to support de novo erythropoiesis in the bone marrow, thus building up iron overload in MDS. Therefore, correction of unbalanced iron absorption by induction of hepcidin synthesis or supplementation of hepcidin mimetics is evaluated as an attractive therapeutic approach to normalize the dysregulated iron metabolism in MDS.

The requirement of chronic transfusion therapy in MDS patients often results in secondary iron overload with eventual life-threatening consequences in this patient population. While transfusion dependency by itself is a negative prognostic factor reflecting poor bone marrow function, the ensuing transfusional iron overload has an additional dose-dependent negative impact on the survival of patients with lower risk MDS. Recent data suggest in fact that markers of iron overload portend a relatively poor prognosis, and retrospective analysis demonstrates that iron chelation therapy is associated with prolonged survival in transfusion-dependent MDS patients.

Diagnosis of suspected MDS is based on clinical and hematological analysis, complemented by genetic analysis for possible genetic abnormalities. So far treatment of MDS includes supportive care, drug therapy and hematopoietic stem cell transplantation. Supportive care may include intermittent or regular blood transfusions, medications to increase the making of red blood cells including erythropoietin-stimulating agents, and antibiotics. Known drugs used in the treatment of MDS include lenalidomide, antithymocyte globulin, and azacitidine. Chemotherapy followed by a stem-cell transplant from a donor is a further treatment option for MDS patients.

Since iron accumulation is an early event in a subset of MDS patients with potentially detrimental effects, and iron chelators often show unwanted side effects, including gastrointestinal symptoms, novel approaches are needed to handle the iron overload condition associated with MDS and implement the currently available therapeutic strategies, with the aim to improve the quality of life and prognosis of this patient population as well as delay the leukemic evolution of the disease. As MDS mainly affect the elderly population, the majority of patients cannot tolerate intensive therapeutic approaches such as allogeneic hematopoietic stem cell transplantation. Also the burden of regular blood transfusions is difficult for elderly patients. New treatment approaches avoiding the disadvantages of the available treatment methods are therefore required.

In a new therapy approach Luspatercept, a recombinant engineered fusion protein that binds transforming growth factor β superfamily ligands to reduce SMAD2 and SMAD3 signaling, showed promising results in a phase 2 study *(*P. Fenaux et al.: "Luspatercept in Patients with Lower-Risk Myelodysplastic Syndromes"; N Engl J Med, 382: 140-151, 2020*).*

Luspatercept is administered parenterally. Luspatercept and its use in the treatment of symptoms of beta-thalassemia, including defective red blood cell production in the marrow and ineffective erythropoiesis, is described e.g. in WO2016183280.

Parenteral administration of drugs usually requires medical attendance, which may increase treatment costs and may influence patient compliance putting additional burden on the patient. Oral drug administration offers advantages over parenteral administration, such as the ease of administration by patients, in particular elderly patients, high degree of flexibility on dosages and formulation, cost-effectiveness, less sterility constraints and risk of infection, injection site reaction and anti-drug antibodies generation.

Considering the significant life-threatening situation of patients suffering from MDS it is apparent that new and improved treatment options are required which achieve increased survival and better quality of life for patients affected by MDS.

Besides the described treatment with Luspatercept, MDS is hitherto conventionally treated with regular blood transfusions (RBC transfusions) accompanied by regular co-treatment with iron chelating compounds which aims at the constant removal of excess iron resulting from the secondary iron overload caused by the regular blood transfusions.

Established drugs used in chelation therapy include deferoxamine (also known as desferrioxamine B; or Desferal^{®}). Two newer drugs for iron chelation therapy, licensed for use in patients receiving regular blood transfusions to treat thalassemia, resulting in the development of iron overload, are deferasirox (also known as Exjade^{®}) and deferiprone (also known as Ferriprox^{®}).

WO2013/086312 A1 describes oral formulations, including desazadesferrithiocin polyether (DADFT-PE) analogues for treating iron overload, such as transfusion dependent hereditary and acquired anemias, via iron chelation as the underlying mechanism of action.

The disadvantage of treating MDS with regular blood transfusions is the continuing need of regular transfusion and regular removal of the excess iron by chelation therapy for the patients. Further, the established drugs for iron chelation therapy are known to exhibit a toxic potential, which becomes potentially problematic in prolonged administration due to long-term need of transfusion therapy.

Low molecular weight compounds having activity as ferroportin inhibitors are described in the international applications WO2017/068089 and WO2017/068090. Further, international application WO2018/192973 relates to specific salts of selected ferroportin inhibitors described in WO2017/068089 and WO2017/068090. The ferroportin inhibitors described in said three international applications overlap with the compounds according to formula (I) used in the present application. Therein a reference to the potential treatment of MDS is just generally mentioned in a list of possible indications without providing any data. The unpublished international application PCT/EP2020/070391 describes the use of a selected group of ferroportin inhibitors in the treatment of transfusion dependent thalassemia.

Manolova Vania et al. "Oral ferroportin inhibitor ameliorates ineffective erythropoiesis in a model of [beta]-thalassemia", The Journal of Clinical Investigation, Vol. 130, No.1, 02.01.2020, pages 491-506, XP055844753, describe experimental studies carried out with a selected oral ferroportin inhibitor compound (VIT-2763, corresponding to Example Compound 127 of the present application), showing that VIT-2763 improves ineffective erythropoiesis and ameliorated anemia, and prevents liver iron loading in a mouse model of β-thalassemia. The paper speculates about a potential (expected) effectiveness of VIT-2763 in correcting ineffective erythropoiesis and iron overload in a range of diseases, including among other the potential effectiveness in ameliorating myeloproliferative/myelodysplastic disorder, such as MDS.

Frank Richard et al: "Oral ferroportin inhibitor VIT-2763: First-in-human, phase 1 study in healthy volunteers", American Journal Of Hematology, Vol. 95, No. 1, 19.11.2019, pages 68-77, XP055657378, presents results from a first-in-human phase 1 study, evaluating the safety, tolerability, pharmacokinetics etc. of the oral ferroportin inhibitor compound VIT-2763 in healthy volunteers. Similar as the above cited paper of Manolova et al. (2020), this paper also speculates in the discussion part about the potential of VIT-2763, due to its ability to restrict iron absorption, to improve erythropoiesis and anemia in patients with ineffective erythropoiesis such as in MDS.

The underlying pathogenesis of MDS and of β-thalassemia (bthal) differs significantly. Significant differences in the mechanisms of ineffective erythropoiesis in bthal versus MDS exist with several aspects being unique to MDS and absent in bthal.

### Ineffective Erythropoiesis in MDS vsβ-Thalassemia:

Ineffective erythropoiesis is a hallmark of other diseases, such as thalassemia. Ineffective erythropoiesis develops under conditions in which erythroid progenitor precursors either fail to mature, die in the process of becoming erythrocytes, or develop into erythrocytes that are abnormal and die prematurely. Although thalassemia and MDS both display ineffective erythropoiesis, the underlying molecular mechanisms differ.

In β-thalassemia, ineffective erythropoiesis is characterized by expansion, limited differentiation, and premature death of erythroid precursors, a process mediated by factors involved in cell cycle, iron intake, and heme synthesis. Specifically, the imbalance in the production of α- and β-globin chains leads to an excess of heme and α-globin elements accumulating as hemichromes. Hemichromes are toxic aggregates that increase oxidative stress and cause cell death due to the presence of reactive iron moiety. Hemichromes precipitate on red blood cell (RBC) membranes, causing changes in membrane structure, inducing lipid peroxidation, and leading to the exposure of the anionic phospholipids that together result in premature RBC clearance from circulation. In β-thalassemia iron restriction in erythroid precursors acts as a compensatory mechanism, whereby reduced cellular iron results in decreased heme synthesis and fewer hemichromes. The delivery of smaller amounts of iron to more erythroid precursors leads to decreased mean cellular hemoglobin (MCH) and fewer hemichromes. Because hemichromes and ROS cause ineffective erythropoiesis in β-thalassemia, iron restriction and decreased erythroid iron intake results in more effective erythropoiesis, normalizes RBC structure and lifespan, increases circulating Hb, and reverses splenomegaly. Thus, the use of drugs that decrease iron uptake from the diet improves erythropoiesis in β-thalassemia.

Although ineffective erythropoiesis is characterized by erythropoietin-driven expansion of early-stage erythroid precursors, associated with apoptosis of erythroid precursors in both β-thalassemia and MDS, erythron expansion is more severe in β-thalassemia and the cellular and molecular mechanisms underlying ineffective erythropoiesis and its aggravation by iron excess are different in β-thalassemia and MDS. In contrast with β-thalassemia where iron is central to disease pathogenesis through hemichrome deposition, MDS is a HSC disease, potentially aggravated - although not directly driven - by iron, and hallmarked by both ineffective erythropoiesis and hematopoieisis. While in β-thalassemia ineffective erythropoiesis is due to premature death of erythroid precursors due to hemichrome formation, in MDS it has been attributed to the differentiation arrest and increased apoptosis of erythroid precursors induced by genetic lesions originating in HSPCs, as well as excessive proinflammatory cytokines and immune disorders in the bone marrow niche, and is independent from hemichromes, which are not generated in MDS.

Thus, iron excess likely aggravates and its restriction improves these mechanisms as described below:

### (1) Contribution of Iron to Ineffective Erythropoiesis in MDS:

Due to the lack of hemichrome formation, the way iron contributes to ineffective erythropoiesis in MDS is different than in β-thalassemia.
- Iron directly impact HSPCs, likely contributing to HSPC exhaustion and thus ineffective erythropoiesis. ROS are deeply entangled with hematopoiesis: while a certain amount of ROS is critical for the coordinated proliferation and differentiation of HSPCs, excessive ROS lead to higher stem cell turn-over and ultimately HSC exhaustion. Thus, HSC exposure to excess iron in the bone marrow niche promotes ROS formation in HSCs, inducing apoptosis in hematopoietic precursors and contributing to ineffective erythropoiesis.
- Iron directly affects the erythroid lineage. Exposure of erythroid precursors to elevated iron induces dysplastic changes and significantly impairs erythroblast differentiation and RBC maturation, causing an overall reduction of burst-forming unit colonies formation and erythroblast apoptosis. These events are reversed by chelation and anti-oxidant agents. Consistent with these observations, HSCs from iron-treated MDS animal and from MDS patients with moderately elevated serum ferritin (>250 µg/l) show impaired proliferation - exclusively in the erythroid lineage. Recent findings suggest that intracellular oxidative stress impairs erythroid development, which can be actively improved by modulation of ferroportin expression on these cells. The enhanced sensitivity of erythroid precursors to iron toxicity might be due to the direct effect of labile iron exposure and/or mitochondrial iron retention, especially in MDS-RS (MDS with ring sideroblasts). In MDS-RS, erythroid precursors accumulate iron in mitochondria (appearing as ring sideroblasts). As a result of the mitochondrial iron retention, iron incorporation into heme is reduced, which contributes to oxidative stress and hypoxia, further fueling the expanded but ineffective erythropoiesis in MDS. Iron in ring sideroblasts plausibly deposits in mitochondrial ferritin, whose levels have been correlated with early apoptosis of MDS-RS erythroblasts. Overall, this suggests that iron excess exacerbates ineffective erythropoiesis through the aggravation of the differentiation defect and apoptosis propensity of MDS erythroid precursors.

### (2) Contribution of Iron to Leukemic Progression:

In addition to ineffective erythropoiesis, in MDS, the presence of labile plasma iron and the associated increase in labile cellular iron and production of ROS have been postulated to play a role in disease pathogenesis through increased apoptosis rate and genomic instability of HSPCs, alterations of the bone marrow microenvironment, and disease progression towards acute myeloid leukemia (AML) with MDS-related features. ROS formation and markers of oxidative DNA damage are elevated and further exacerbated by transfusional iron overload in the bone marrow of MDS patients and corrected by iron chelation therapy. Iron excess has also been implicated in the induction of epigenetic abnormalities and telomere erosion. Overall iron-induced oxidative stress, DNA damage and telomere shortening likely contribute to bone marrow mutagenesis, underscoring iron as a potential additional driver of genomic instability and malignant transformation in MDS. Despite unable to trigger per se stem cell leukemic transformation, iron overload might accelerate leukemic progression by mediating genotoxic stress in highly proliferating HSPCs. Moreover, the exhaustion of normal HSCs due to their exit from quiescence induced by iron-driven ROS elevation, likely contributes to the selective expansion of the MDS clone. This indicates that iron by promoting malignant transformation and normal HSC exhaustion, might play a role in clonal expansion and myeloid leukemia progression.

### (3) Contribution of Iron to Deranged Bone Marrow Microenvironment:

An abnormal bone marrow microenvironment plays a critical role in MDS pathogenesis and the evolution of lower-risk MDS to a more aggressive disease. Due to the key function of the bone marrow microenvironment in the maintenance, self-renewal and differentiation of HSCs, its alterations have been implicated in hematopoiesis impairment as well as progenitor cell apoptosis and dysplasia. Iron likely contributes to the decreased survival and functional impairment of multiple cell types within the bone marrow microenvironment, including mesenchymal stromal cells (MSC), bone cells, immune cells and vascular endothelial cells. Iron-driven alterations of the mesenchymal cell compartment influence their supporting function for hematopoiesis. Indeed, the expression of several adhesion molecules and cytokine secretion is altered in bone marrow stroma cells under iron overload conditions impairing their capacity to support hematopoietic cells growth. Moreover, the immunomodulatory role of iron and transfusions through the induction of altered cell functions and cytokine production in immune cells likely play a role in bone marrow niche dysfunction and deranged hematopoiesis in MDS patients who often present with a pro-inflammatory niche.

### (4) Contribution of Iron Overload to Organ Toxicity:

Beside the bone marrow, an altered iron metabolism can also impact other organs. Similarly to β-thalassemia, in MDS, iron overload due to multiple transfusions has been demonstrated to be toxic to various organs as liver, heart, pancreas, thyroid and pituitary gland leading to an increased morbidity and mortality.

The inventors of the present invention surprisingly found, that ferroportin inhibitor compounds as defined herein not only act to block ferroportin, but even further improves the following aspects in steady-state MDS:
- ineffective erythropoiesis
- survival of HSCs
- myeloid expansion
- inflammation in the bone marrow microenvironment
- tissue iron overload

While ineffective erythropoiesis is improved by ferroportin inhibitor-mediated iron restriction in both, β-thalassemia and MDS, the underlying mechanism is centered on the reduction of hemichrome formation in erythroid precursors in β-thalassemia, while in MDS it is centered on a multifactorial amelioration of quality and quantity of HSCs and erythroid progenitors (e.g. reduced apoptosis, improved maturation) due to decreased ROS, upon reduced iron availability.

Improvements of aspects unique to MDS, including limited exhaustion of the HSC pool, reduced myeloid expansion and leukemic progression, and decreased inflammation in the bone marrow microenvironment, surprisingly show the effectiveness also in MDS, being based on additional and different mode of action compared to β-thalassemia.

Importantly, the findings of the inventors of the present application prove that enhanced iron absorption is of pathological relevance and its inhibition via the ferroportin inhibitors described herein of therapeutic benefit in steady-state MDS, through the modulation of underlying pathophysiologic mechanisms, including ineffective erythropoiesis, HSC exhaustion and myeloid clone expansion.

### OBJECT OF THE INVENTION

Note that the references to the methods of treatment by therapy in this description are to be interpreted as references to compounds, pharmaceutical compositions and medicaments of the present invention for use in those methods.

The object of the present invention is to provide a new method for treating myelodysplastic syndromes (MDS). A particular object of the present invention can be seen in providing novel drug compounds for effectively treating MDS and the symptoms and pathological conditions associated therewith or ameliorating the burden connected with the conventional MDS treatment methods. In particular, novel drug compounds for treating MDS and the symptoms and pathological conditions associated therewith or for ameliorating the burden connected with the conventional MDS treatment methods using improved administration routes, such as in particular oral administration should be provided to simplify administration, reduce side-effects resulting from parenteral administration, enhance patient compliance, safe treatment costs and reduce the treatment burden for the patients. In a further aspect an object of the invention can be seen in providing compounds for treating MDS and the symptoms and pathological conditions associated therewith, which are easier and cheaper to prepare than drugs based on recombinant engineered proteins or genetically engineered drug compounds.

### DESCRIPTION OF THE INVENTION

The inventors of the present invention surprisingly found that a compound of the general formula (I) as defined herein, which acts as ferroportin inhibitor (Fpnl), can be used for treating MDS and the symptoms and pathological conditions associated therewith, such as in particular defective red blood cell production in the bone marrow, ineffective hematopoiesis such as in particular ineffective erythropoiesis, low hemoglobin levels / anemia, iron overload and multiple organ dysfunction, liver and kidney iron loading and cardiac iron overload. In a further aspect, the ferroportin inhibitor compound as defined herein can be used for reducing bone marrow immature cells and myeloblasts in MDS patients and thus myeloid expansion, possibly preventing or delaying leukemia evolution in MDS patients, for reducing the production of inflammatory cytokines as TNFα and IL-1β by macrophages, and/or improving the bone marrow microenvironment. In particular, the new and surprising results showing delay in leukemia evolution offer a new approach of treating leukemia with the ferroportin inhibitor compound as defined herein.

Accordingly, a first aspect of the present disclosure relates to compounds according to formula (I) below for use in the treatment of myelodysplastic syndromes (MDS): 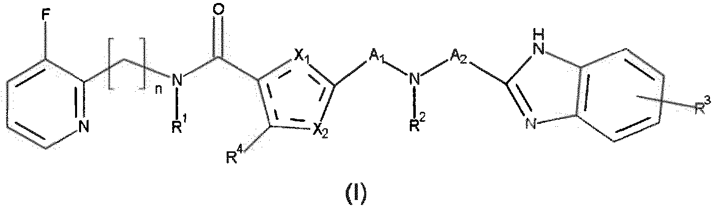 wherein
X¹ is N or O; and
X² is N, S or O;
with the proviso that X¹ and X² are different;
R' is selected from the group consisting of
   - hydrogen and
   - optionally substituted alkyl;
n is an integer of 1 to 3;
A' and A² are independently selected from the group of alkanediyl
R² is
   - hydrogen, or
   - optionally substituted alkyl;
   or
A' and R² together with the nitrogen atom to which they are bonded form an optionally substituted 4- to 6-membered ring;
R³ indicates 1, 2 or 3 optional substituents, which may independently be selected from the group consisting of
   - halogen,
   - cyano,
   - optionally substituted alkyl,
   - optionally substituted alkoxy, and
   - a carboxyl group;
R⁴ is selected from the group consisting of
   - hydrogen,
   - halogen,
   - C₁-C₃-alkyl, and
   - halogen substituted alkyl;
including also pharmaceutically acceptable salts, solvates, hydrates and polymorphs thereof.

The compound according to the present invention is the compound as defined in claim 1. Indication

The present invention relates to the selected medical use of the compound of claim 1 and its salts, solvates, hydrates and polymorphs, for the treatment of MDS.

The treatment of myelodysplastic syndromes (MDS) and/or the symptoms associated therewith comprises the treatment of ineffective hematopoiesis, in particular ineffective erythropoiesis.

The treatment of MDS and/or the symptoms associated therewith further comprises ameliorating, preventing or delaying leukemia evolution, reducing bone marrow immature cells and myeloid expansion, reducing the production of inflammatory cytokines as TNFα and IL-1β by macrophages, and/or improving the bone marrow microenvironment.

As mentioned above, several subtypes of MDS are classified in the International Prognostic Scoring System (IPSS) and the Revised IPSS (IPSS-R). Lower-risk MDS are defined according to the IPSS as being of low or intermediate 1 risk, or according to the Revised IPSS [IPSS-R] as being of very low, low, or intermediate risk.

The present invention relates to the compound of claim 1 or its salts, solvates, hydrates and polymorphs, for the treatment of MDS, wherein the MDS patients are selected from individuals suffering from very-low-risk, low-risk or intermediate-risk myelodysplastic syndromes according to the IPSS / IPSS-R scoring system. The treatment of lower-risk MDS (IPSS) is preferred.

Further, genetic MDS subtypes are defined, such as MDS with isolated del(5q) or MDS with SF3B1 mutation. The report paper of Malcovati et al. (2020), cited above, further defines MDS subtypes and diagnostic criteria for MDS entities, listed in the following Table 1:

| Name | | Dysplastic lineages | Cytopenias | RS (%)* | BM and PB blasts (%) | Cytogeneticst |
|---|---|---|---|---|---|---|
| MDS-SLD | | 1 | 1 or 2 | <15/<5‡ | BM <5, PB <1, no Auer rods | Any, unless fulfills all criteria for MDS with isolated del(5q) |
| MDS-MLD | | 2 or 3 | 1-3 | <15/<5‡ | BM <5, PB <1, no Auer rods | Any, unless fulfills all criteria for MDS with isolated del(5q) |

| **MDS-RS** | | | | | | |
|---|---|---|---|---|---|---|
| | MDS-RS-SLD | 1 | 1 or 2 | ≥15/≥5‡ | BM < 5, PB <1, no Auer rods | Any, unless fulfills all criteria for MDS with isolated del(5q) |
| | MDS-RS-MLD | 2 or 3 | 1-3 | ≥15/≥5‡ | BM <5, PB <1, no Auer rods | Any, unless fulfills all criteria for MDS with isolated del(5q) |
| | MDS with isolated del(Sq) | 1-3 | 1-2 | None or any | BM <5, PB <1, no Auer rods | Del(5q) alone or with 1 additional abnormality except -7 or del(7q) |

| **MDS-EB** | | | | | | |
|---|---|---|---|---|---|---|
| | MDS-EB-1 | 0-3 | 1-3 | None or any | BM 5-9 or PB 2-4, no Auer rods | Any |
| | MDS-EB-2 | 0-3 | 1-3 | None or any | BM 10-19 or PB 5-19 or Auer rods | Any |

| **MDS-U** | | | | | | |
|---|---|---|---|---|---|---|
| | 1% blood blasts | 1-3 | 1-3 | None or any | BM <5, PB = 1,§ no Auer rods | Any |
| | SLD and pancytopenia | 1 | 3 | None or any | BM <5, PB <1, no Auer rods | Any |
| | Defining cytogenetic abnormality | 0 | 1-3 | <15∥ | BM <5, PB <1, no Auer rods | MDS-defining abnormality |
| | Refractory cytopenia of childhood | 1-3 | 1-3 | None | BM <5, PB <2 | Any |

| | | | | | | |
|---|---|---|---|---|---|---|
| BM, bone marrow; MDS-EB, MDS with excess blasts; MDS-U, MDS, unclassifiable; PB, peripheral blood. *RS as percentage of marrow erythroid elements. †Cytogenetics by conventional karyotype analysis. ‡If SF3B1 mutation is present. §One percent PB blasts must be recorded on ≥2 separate occasions. ∥Cases with ≥15% RS by definition have significant erythroid dysplasia and are classified as MDS-RS-SLD. | | | | | | |

A further aspect of the present invention relates to the compound of claim 1, or its salts, solvates, hydrates and polymorphs, for the treatment of MDS, selected from one of the MDS entities defined in the above table 1.

In a further aspect of the invention the MDS-patients to be treated are selected from one or more of the following patient groups, wherein the individuals are characterized by one or more of the following:
- suffering from myelodysplastic syndromes with ring sideroblasts (RS) according to World Health Organization criteria, characterized by either 215% ring sideroblasts, or ≥5% ring sideroblasts if an SF3B1 mutation is present, or with <5% bone marrow blasts:
- suffering from myelodysplastic syndromes having erythropoietin levels above 200 U per liter;
- suffering from erythroid dysplasia;
- suffering from cytopenia, in particular peripheral cytopenia;
- bone marrow blasts < 5%;
- peripheral blood blasts < 1%;
- suffering from myelodysplastic syndromes with reduced or lack of response to erythropoiesis-stimulating agents;
- suffering from myelodysplastic syndromes with chromosome 5q deletion (del[5q]);
- SF3B1-mutant patients;
- patients with significantly down-regulated PPOX and/or ABCB7 genes compared to healthy individuals;
- being transfusion-dependent or receiving regular red blood cell transfusions of ≥ 2 units per 8 weeks.

In one aspect the present invention relates to the compound of claim 1, or its salts, solvates, hydrates and polymorphs, for the treatment of transfusion-independent MDS.

In a further aspect the present invention relates to the compound of claim 1, or its salts, solvates, hydrates and polymorphs, for the treatment of transfusion-dependent MDS, i.e. to the treatment of MDS according to the present invention, wherein the selected patient group is characterized by requiring regular blood transfusions or being transfusion-dependent patients. Such regular blood transfusion or transfusion-dependency is characterized by
a) repeated blood transfusions of equal red blood cell (RBC) units in varying subsequent time intervals or
b) repeated blood transfusions of equal RBC units in equal subsequent time intervals or
c) repeated blood transfusions of varying RBC units in equal subsequent time intervals or
d) repeated blood transfusions of varying RBC units in varying subsequent time interval.

The term "treat", "treatment" or "treating" in the context of the use of the present invention includes amelioration of at least one symptom or pathological condition associated with MDS. Non-limiting examples of symptoms or pathological conditions associated with MDS include defective red blood cell production in the marrow, ineffective hematopoiesis such as in particular ineffective erythropoiesis, deficient hemoglobin levels, multiple organ dysfunction, iron overload, anemia, liver iron loading and cardiac iron overload, as well as the symptoms described above and in the example below.

The term "treat", "treatment" or "treating" in the context of the present invention further includes prophylaxis, e.g. by administering the compounds of the present invention prior to or accompanying blood transfusion in transfusion-dependent MDS patients to prevent or at least attenuate occurrence of transfusion-caused pathological conditions.

Patients with MDS may have severe iron overload due to regular blood transfusion (BT). The major goals of blood transfusion therapy in the treatment of MDS are to correct the anemic condition and suppress erythropoiesis. This is considered to be accomplished at an Hb level of ≥ 9 g/dL. Therefore, in a further aspect of the treatment of MDS patients, the administration of the ferroportin inhibitor compounds of formula (I) helps to prevent intestinal iron absorption during the intervals between transfusions, which helps to reduce further iron loading in MDS patients.

It has been observed that MDS patients have elevated levels of non-transferrin bound iron (NTBI). NTBI is released by macrophages recycling damaged RBCs resulting from immature RBC formation in the bone marrow and/or contained in transfused RBC units and triggers oxidative stress, vascular damage and organ iron overload *(*Baek J. H. et al, "Iron accelerates hemoglobin oxidation increasing mortality in vascular diseased guinea pigs following transfusion of stored blood." JCI Insight, 2017; 2(9*)).*

The inventors of the present invention found that the compound of the present invention is particularly suitable for the treatment of MDS by improving ineffective erythropoiesis through restriction of iron excess mediated by the compound. It is further assumed that the compound of the present invention is particularly suitable for the treatment of MDS by limiting reactive oxygen species (ROS) in erythroid precursors and thereby improving erythropoiesis in patients suffering from MDS. As a result, more RBCs with extended life-span ameliorate anemia in MDS patients and improve tissue oxygenation. In MDS the compound of claim 1 further efficiently diminishes elevated NTBI levels, which helps to prevent the occurrence of pathological conditions deriving therefrom, such as e.g. liver, kidney and cardiac iron overload and thus organ dysfunction and other diseases.

NTBI, which encompasses all forms of serum iron that are not tightly associated with transferrin or other molecules, is chemically and functionally heterogeneous. LPI (Labile Plasma Iron) represents a component of NTBI that is both redox active and chelatable, capable of permeating into organs and inducing tissue iron overload. The compounds of the formula (I) have the potential to efficiently diminish elevated NTBI and thus LPI levels in MDS.

The following parameters can be determined to evaluate the efficacy of the compounds of the present invention in the medical use of treating MDS: serum iron, NTBI levels, LPI (Labile Plasma Iron) levels, erythropoietin, TSAT (transferrin saturation), Hb (hemoglobin), Hct (haematocrit), MCV (Mean Cell Volume), MCH (Mean Cell Hemoglobin), RDW (Red Blood Cell Distribution Width) and reticulocyte numbers, complete blood counts, myeloblasts in the bone marrow and peripheral blood, spleen weight, erythropoiesis in bone marrow and spleen, liver, spleen and kidney iron content. The determination can be carried out using conventional methods of the art, in particular by those described below in more detail. The compound of the present invention is suitable to improve at least one of these parameters.

As explained by *Patel et al. (2012; cited above)* in normal physiological conditions the level of transferrin is sufficient for complete scavenging of the absorbed and recycled iron, ensuring the absence of NTBI and accordingly NTBI levels in normal healthy individuals do not exceed 0.1 µmol/L and are mostly undetectable by most common methods. In the absence of transferrin NTBI levels up to 20 µmol/L were reported and in the presence of insufficient transferrin or highly saturated transferrin NTBI levels up to 10 µmol/L have been found. However, as described by *Patel et al. (2012)* and *Brissot et al. (2012)* the determination strongly depends from the applied method and assays used and the technical difficulties resulting from the determination of heterogeneous chemical forms of circulating NTBI must be taken into account. For example, fluorescent measurements with a repeatable accuracy down to 0.1 µmol/L have been described by Hider et al. (2010) cited by *Brissot et al. (2012).* According to *Patel et al. (2012; Table* 1) elevated NTBI levels in clinical iron overload conditions range between 0.25 to 4.0 µmol/L (with varying accuracy and varying determination methods). Considering this, in the sense of the present invention NTBI levels are considered as elevated if detectable with the known methods (e.g. those described in *Patel* et *al. (2012)* or in *Brissot et al. (2012),* preferably when exceeding 0.1 µmol/L.

In a particular aspect, the treatment of MDS according to the present invention results in reduced NTBI levels in a patient by at least 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95%, or at least 100%, determined at any time point within a time period of up to 72 hours, up to 60 hours, up to 48 hours, up to 36 hours, up to 24 hours, or up to 12, 8, 6, 5, 4, 3, 2, 1 and 0.5 hours following the administration and as compared to the NTBI levels in the patient determined at any time point within 0.5, 1, 2, 3, 4, 5, 6, 8, 12, 24, 36, or 48 hours, or up to < 1 week prior to the commencement of treatment of the invention. NTBI can be determined according to assays described in the Examples below.

In a particular aspect, the treatment of MDS according to the present invention results in reduced LPI levels in a patient by at least 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95%, or at least 100%, determined at any time point within a time period of up to 72 hours, up to 60 hours, up to 48 hours, up to 36 hours, up to 24 hours, or up to 12, 8, 6, 5, 4, 3, 2, 1 and 0.5 hours following the administration and as compared to the total LPI levels in the patient determined at any time point within 0.5, 1, 2, 3, 4, 5, 6, 8, 12, 24, 36, or 48 hours, or up to < 1 week prior to the commencement of treatment of the invention. LPI can be determined according to an assay described in the Examples below.

Reactive oxygen species (ROS) lead to a shortened lifespan of RBCs, anemia and tissue hypoxia. The effect of the compounds of the present invention on ROS levels in RBCs can be monitored by commercially available far-red or green emitting ROS-sensitive sensor, e.g. as described in the Examples below.

In a further aspect, the treatment of MDS according to the present invention results in reduced ROS levels in RBCs of the patients by at least 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95%, or at least 100%, determined at any time point within a time period of up to 5 days, up to 6 days, up to 7 days, up to 8 days, up to 9 days, up to 10 days, up to 11 days, up to 12 days, up to 13 days, up to 14 days, up to 15 days, up to 16 days, up to 17 days, up to 18 days, up to 19 days, up to 20 days, up to 21 days and up to 1 month following the first administration and/or following an ischemic event and as compared to the ROS levels in RBCs of the patient determined at any time point within 12 hours, 24 hours, 36 hours, 48 hours, 1 week, 2 weeks, 3 weeks, or 4 weeks prior to the commencement of treatment of the invention. ROS levels in RBCs can be determined according to an assay described in the Examples below.

As explained above, the reduction of elevated NTBI and LPI levels helps to decrease liver, kidney and myocardial iron concentration.

Accordingly, in a further aspect, the treatment of MDS according to the present invention may result in a decrease in liver iron concentration in the patient by at least 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95%, or at least 100%, determined at any time point within a time period of up to one week, up to 2 weeks, up to 3 weeks, up to 4 weeks, up to 3 months following the first administration and as compared to the levels of liver iron concentration in the patient determined at any time point within 1 week, 2 weeks, 3 weeks, or 4 weeks prior to the commencement of treatment of the invention. Liver iron concentration can be determined according to an assay described in the Examples below.

Accordingly, in a further aspect, the treatment of MDS according to the present invention may result in a decrease in kidney iron concentration in the patient by at least 1%, 2%, 3%, 4%, 5%, **10%, 15%,** 20%, 25%, 30%, 35%, **40%, 45%, 50%, 55%, 60%, 65%, 70%, 75%, 80%, 85%,** 90%, 95%, or at least 100%, determined at any time point within a time period of up to one week, up to 2 weeks, up to 3 weeks, up to 4 weeks, up to 3 months following the first administration and as compared to the levels of kidney iron concentration in the patient determined at any time point within 1 week, 2 weeks, 3 weeks, or 4 weeks prior to the commencement of treatment of the invention. Kidney iron concentration can be determined according to an assay described in the Examples below.

In a further aspect, the treatment of MDS according to the present invention may result in a decrease in myocardial iron concentration in the patient by at least 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95% or at least 100 %, determined at any time point within a time period of up to one week, up to 2 weeks, up to 3 weeks, up to 4 weeks, up to 3 months following the first administration and as compared to myocardial iron concentration in the subject determined at any time point within 1 week, 2 weeks, 3 weeks, or **4** weeks prior to the commencement of treatment of the invention. Myocardial iron concentration can be determined according to an assay described in the Examples below.

In a further aspect, the treatment of MDS according to the present invention may result in an improvement of at least one of the parameters Hb, Hct, RBC counts, MCV, MCH, RDW, and reticulocyte numbers in the patient by at least 5%, 10%, 15%, 20%, 25%, 30%, 35%, 40%, 45%, 50%, 55%, 60%, 65%, 70%, 75%, 80%, 85%, 90%, 95% or at least 100 %, determined at any time point within a time period of up to one week, up to 2 weeks, up to 3 weeks, up to 4 weeks, up to 3 months following the first administration and as compared to the respective parameter in the subject determined at any time point within 1 week, 2 weeks, 3 weeks, or 4 weeks prior to the commencement of treatment of the invention. Said parameters can be determined according to conventional methods.

In a further aspect, the treatment of MDS according to the present invention may result in an erythroid response, which may comprises a reduction in transfusion burden in the patient by at least 33 %, preferably by at least 50%. In principle, the erythroid response may comprises a reduction in transfusion burden in the patient by at least 10%, 15%, 20%, 25%, 30%, 33%, 35%, 40%, 45%, 50%, 55%, 60%, 65%, 70%, 75%, 80%, 85%, 90%, 95%, 98%, or 100%. In a further aspect, the treatment of MDS according to the present invention may result in an erythroid response, which may comprise a reduction in transfusion burden in the patient by at least 10%, 15%, 20%, 25%, 30%, 33%, 35%, 40%, 45%, 50%, 55%, 60%, 65%, 70%, 75%, 80%, 85%, 90%, 95%, 98%, or 100% for at least 2 weeks, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 5 months, 6 months, 7 months, 8 months, 9 months, 10 months, 11 months, 12 months, up to 18 months, up to 24 months or even beyond up to transfusion independence. In a further aspect, the treatment of MDS according to the present invention may result in an erythroid response, which may comprise a reduction of red blood cell transfusion in the patient by at least 1, 2, 3, 4 or more red blood cells units. In a further aspect, the treatment of MDS according to the present invention may result in an erythroid response, which comprises a reduction of red blood cell transfusion in the patient by at least 1, 2, 3, 4 or more red blood cells units for at least 2 weeks, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 5 months, 6 months, 7 months, 8 months, 9 months, 10 months, 11 months, 12 months, up to 18 months, up to 24 months or even beyond up to independence of transfusion of red blood cell units. It is also possible that the erythroid response comprises one or more of the aforesaid improvements. Erythroid response can be determined as described in the Examples below.

Therein, one unit of red blood cells refers to a quantity of packed red blood cells derived from approximately 200-500 mL of donated blood. Usually, blood transfusions are adjusted depending on the age, severity of the disease and the patient's starting blood parameters. Guidelines for choosing the amount of blood transfusions recommend e.g.:

| | | **HAEMATOCRIT OF DONOR RED CELLS** | | | |
|---|---|---|---|---|---|
| | | **50%** | **60%** | **75%** | **80%** |
| **Target increase in haemoglobin level** | 2 g/dl | 12 ml/kg | 10 ml/kg | 8 ml/kg | 7.5 ml/kg |
| | 3 g/dl | 18 ml/kg | 15 ml/kg | 12 ml/kg | 11.2 ml/kg |
| | 4 g/dl | 24 ml/kg | 20 ml/kg | 16 ml/kg | 15 ml/kg |

An individual blood transfusion volume can further be calculated with the following formula: 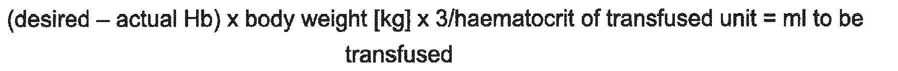 (desired - actual Hb) x body weight [kg] x 3/haematocrit of transfused unit = ml to be transfused

According to the recommended transfusion scheme for MDS the equivalent of 100 to 200 ml of pure red blood cell (RBC) per kg body weight per year are transfused.

In a further aspect, the treatment of MDS according to the present invention may result in a reduction of transfusion burden in the patient compared to the transfusion burden for the patient within 1 week, 2 weeks, 3 weeks or 4 weeks, 2 months, 3 months, 4 months, 6 months, 8 months, 9 months, 12 months, 24 months, prior to the commencement of treatment of the invention.

In a further aspect, the treatment of MDS according to the present invention may achieve that the MDS patient treated according to the method of the present invention does not require red blood cell transfusion for at least 2 weeks, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 5 months, 6 months, 7 months, 8 months, 9 months, 10 months, 11 months, 12 months, 18 months, 24 months or even longer up to independence from red blood cell transfusions after treatment.

In a further aspect, the treatment of MDS according to the present invention may result in reduced daily iron chelation therapy in transfusion-receiving MDS patients, such as, for example, a decrease in the dose or frequency of one or more iron chelation therapeutic agents administered to the patient. Non-limiting examples of iron chelation therapeutic agents include those mentioned above.

In a further aspect, the treatment of MDS according to the present invention may result in a reduction of therapy with erythropoietin stimulating agents, such as erythropoietin (EPO), such as, for example, a decrease in the dose or frequency of erythropoietin stimulating agents administered to the MDS patient.

In a further aspect, the treatment of MDS according to the present invention may result in reduced serum ferritin levels in the patient by at least 5%, 10%, 15%, 20%, 25%, 30%, 35%, 40%, 45%, 50%, 55%, 60%, 65%, 70%, 75%, 80%, 85%, 90%, 95% or at least 100 %, determined at any time point within a time period of up to one week, up to 2 weeks, up to 3 weeks, up to 4 weeks, up to 3 months following the first administration and as compared to the serum ferritin levels in the patient determined at any time point within 1 week, 2 weeks, 3 weeks, or 4 weeks prior to the commencement of treatment of the invention. Serum ferritin levels can be determined according to conventional assays.

In a further aspect, the treatment of MDS according to the present invention may result in a reduction of the symptoms associated with one or more clinical MDS complications. Non-limiting examples of MDS symptoms include pallor, jaundice, fatigue, and clinical complications of chronic red blood cell transfusions, such as, for example hepatitis B virus infection, hepatitis C virus infection and human immunodeficiency virus infection, alloimmunization, and organ damage due to iron overload, such as, for example, liver damage, heart damage and endocrine gland damage.

In a further aspect, the treatment of MDS according to the present invention may result in an improvement in the quality of life in the patients as compared to the quality of life in the patients determined within the 1, 2, 3, or 4 week(s) prior to the commencement of treatment of the invention. The improvement of Quality of life is determined within 3, 6, 9, 12, 15, 18, 21 or 24 months after the commencement of the treatment. Quality of life can be determined according to an assay described in the Examples below.

With the treatment of MDS according to the present invention one or more of the aforesaid improvements can be achieved.

### Patient Group

The present invention relates to the medical use of the compound of claim 1 and its salts, solvates, hydrates and polymorphs, as described herein, for the treatment of MDS, in particular one or more of the MDS entities / subtypes defined above.

In principle, the subjects to be treated in the use according to the invention can be any mammals such as rodents and primates, and in a preferred aspect the medical use relates to the treatment of humans. The subjects suffering from MDS and to be treated with the method according to the invention are also designated as "patients" or "individuals".

In particular, MDS patients to be treated according to the present invention are characterized by the underlying pathophysiologic mechanisms explained above in detail, including suffering from ineffective erythropoiesis, HSC exhaustion and myeloid clone expansion.

The subjects to be treated can be of any age. A preferred aspect of the invention relates to the treatment of elderly people. Accordingly, in a preferred aspect of the invention the subjects to be treated with the new methods described herein are more than 25 years old. In a further aspect of the invention the subjects to be treated with the new methods described herein are 25-30 years old, or greater than 30 years old, such as preferably 25-30 years old, 30-35 years old, 35-40 years old, 40-45 years old, 45-50 years old, 50-55 years old, 55-60 years old, or greater than 60 years old. In the preferred case of treating elderly patients the subjects to be treated with the new methods described herein are 60-65 years old, 65-70 years old, 70-75 years old, 75-80 years old, or greater than 80 years old.

The treatment of elderly patients is particularly preferred due to the significant advantages provided by the treatment with the ferroportin inhibitor compound of the present invention. Said compound can be administered orally, which is advantageous over parenteral administration of the so far available drugs (e.g. Luspatercept). Further, the orally bioavailable ferroportin inhibitor of the present invention turned out to have a moderate bioavailability and half-life in the body and are thus relatively quickly washed out. This leads to less adverse effects and a faster reversibility of the drug, which is of particular importance in the treatment of elderly patients.

The patient group or population suffering from MDS and to be treated with the method according to the invention are selected from subjects (patients) being characterized as defined above. In a further aspect of the invention the patient group or population suffering from MDS to be treated with the method according to the invention are selected from subjects (patients) having elevated NTBI levels. NTBI levels are considered as elevated, if detectable with the known methods as discussed above. Preferably, NTBI levels ≥ 0.1 µmol/L are considered as elevated in MDS patients. More preferably, elevated NTBI levels in MDS patients according to the present invention are NTBI values exceeding the values determined in healthy individuals in the respective determination method as described in *de* Swart et al. "Second international round robin for the quantification of serum non-transferrin-bound iron and labile plasma iron in patients with iron-overload disorders" Haematologica, 2016; 101(1): 38-45*.*

In a further aspect of the invention the patient group or population suffering from MDS and to be treated with the method according to the invention are selected from subjects (patients) having elevated LPI levels. LPI levels are considered as elevated, if detectable with the known methods as discussed above. Preferably, elevated LPI levels in MDS patients according to the present invention are LPI values exceeding the values determined in healthy individuals in the respective determination method as described in de Swart et al. "Second international round robin for the quantification of serum non-transferrin-bound iron and labile plasma iron in patients with iron-overload disorders" Haematologica, 2016; 101(1): 38-45*.*

In a further aspect of the invention the patient group or population suffering from MDS and to be treated with the method according to the invention are selected from subjects (patients) having elevated TSAT levels. Preferably, elevated TSAT levels in MDS patients according to the present invention are TSAT levels exceeding the average "normal" TSAT level determined in healthy individuals in the respective determination method. A TSAT of about 25% is considered as average. However, reference ranges depend on multiple factors like age, sex, race and test devices. Most laboratories define "normal" as max. 30% for female and max. 45% for male persons. Above 50% the risk of toxic non-transferrin bound iron (NTBI) rises exponentially, potentially causing organ damage. The TSAT level can further be used to reflect NTBI indirectly and can therefore be used as a translational marker.

In a further aspect of the invention the patient group or population suffering from MDS and to be treated with the method according to the invention are selected from subjects (patients) with dysfunctional and pro-apoptotic hematopoietic stem and progenitor cells (HSPCs) carrying MDS mutations.

Usually, one or more of the following diagnostic criteria for MDS are applied:
(1) cytopenia as defined by standard hematologic values,
(2) genetic analysis for somatic SF3B1 mutation,
(3) morphologic dysplasia (with or without RS),
(4) bone marrow blasts <5% and peripheral blood blasts <1%,
(5) leukemic progression, development of AML.

One of the most important hematologic values is the hemoglobin level (Hb). Patients suffering from MDS maintain Hb levels between 5 to 10 g/dl. Patients suffering from MDS are usually classified anemic by a Hb level of < 9 g/dL or < 8 g/dL. Hb levels in MDS patients may be as low as 4 to 5 g/dl. Although, international guidelines recommend transfusing patients reaching a hemoglobin range of 9-10 g/dL with the optimal post-transfusion range being 13-14 g/dL, in clinical practice Hb levels > 7 g/dL are usually considered as sufficient without regular transfusion and then under transfusion the usual aim is to maintain patients at hemoglobin levels between 9.5 and 10 g/dL. However, depending on the conditions, patients with a Hb level between 7 and 8 g/dL may be found to need transfusion. Reaching the recommended higher Hb levels of 13-14 g/dL would require an undue increase in transfusion burden. However, the amount of blood required varies greatly between patients and is largely influenced by the patient's weight and targeted hemoglobin level.

Considering this, in a further aspect of the invention the patient group or population suffering from MDS and to be treated with the method according to the invention can be selected from subjects (patients) having hemoglobin (Hb) levels below 8 g/dL.

In a further aspect of the invention the patient group or population suffering from MDS and to be treated with the method according to the invention can be selected from subjects (patients) having an MCV between 50 and 70 fL.

In a further aspect of the invention the patient group or population suffering from MDS and to be treated with the method according to the invention can be selected from subjects (patients) having an MCH between 12 and 20 pg.

In a further aspect the patient group or population suffering from MDS and to be treated with the method according to the invention can be selected from subjects (patients) having one or more of the characteristics comprising a) Hb levels below 8 g/dL, b) MCV between 50 and 70 fL and c) MCH between 12 and 20 pg.

In a further aspect of the invention the patient group or population suffering from MDS and to be treated with the method according to the invention receives regular blood transfusions. However, further clinical symptoms and parameters also play an important role in determining MDS as discussed above in detail.

Regular blood transfusions further mean more than one repeating transfusion of red blood cell (RBC) units within time intervals of at least up to two months or in shorter intervals. The intervals may be of equal length or may vary depending on the individual patient, the course of disease, its severity and the treatment response. Regular blood transfusion may further comprise the repeating transfusion of equal or varying transfusion units at subsequent transfusion time points. Regular blood transfusion may comprise
- repeated blood transfusions of equal RBC units in varying subsequent time intervals or
- repeated blood transfusions of equal RBC units in equal subsequent time intervals or
- repeated blood transfusions of varying RBC units in equal subsequent time intervals or
- repeated blood transfusions of varying RBC units in varying subsequent time intervals.

In a further aspect of the invention regular blood transfusion means transfusion-free periods of not more than 3 months, preferably of not more than 2 months.

In a further aspect of the invention the patient group or population suffering from MDS and to be treated with the method according to the invention are selected from subjects (patients) which require regular iron chelation therapy. Such patient group or population requiring regular iron chelation therapy may further be characterized by one or more of the characteristics defined above.

### Administration Forms

In a further aspect of the invention the treatment of MDS comprises the oral administration of the compound of claim 1, its salts, solvates, hydrates or polymorphs, each as described anywhere herein, to a patient in need thereof. For this purpose, the compound according to the invention is preferably provided in medicaments or pharmaceutical compositions in the form of oral administration forms, including e.g. pills, tablets, such as enteric-coated tablets, film tablets and layer tablets, sustained release formulations for oral administration, depot formulations, dragees, granulates, emulsions, dispersions, microcapsules, microformulations, nanoformulations, liposomal formulations, capsules, such as enteric-coated capsules, powders, microcrystalline formulations, epipastics, drops, ampoules, solutions and suspensions for oral administration.

In a preferred embodiment of the invention the compound according to the invention is administered in the form of a tablet or capsule, as defined above. These may be present, for example, as acid resistant forms or with pH dependent coatings.

Accordingly, a further aspect of the present invention relates to the compound according to the invention, including pharmaceutically acceptable salts, solvates, hydrates and polymorphs thereof, as well as medicaments, compositions and combined preparations comprising the same for the use in the treatment of MDS in the form of oral administration forms.

### Dosing Regimen

A further aspect of the invention relates to the compound according to the invention for the use according to the present invention, wherein the treatment is characterized by one of the following dosing regimens:
In one aspect the compound according to the invention can be administered to a patient in need thereof in a dose of 0.001 to 500 mg, for example 1 to 4 times a day. However, the dose can be increased or reduced depending on the age, weight, condition of the patient, severity of the disease or type of administration. In a further aspect of the invention the compound ) can be administered as a dose of 0.1 mg, 0.2 mg, 0.3 mg, 0.4 mg, 0.5 mg, 0.6 mg, 0.7 mg, 0.8 mg, 0.9 mg, 1 mg, 1.5 mg, 2 mg, 2.5 mg, 3 mg, 3.5 mg, 4 mg, 4.5 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, 200 mg, 205 mg, 210 mg, 215 mg, 220 mg, 225 mg, 230 mg, 235 mg, 240 mg, 245 mg, 250 mg, 255 mg, 260 mg, 265 mg, 270 mg, 275 mg, 280 mg, 285 mg, 290 mg, 295 mg, 300 mg, 325 mg, 350 mg, 375 mg, 400 mg, 425 mg, 450 mg, 475 mg, 500 mg.

Preferred is a dose of between 0.5 to 500 mg, more preferred between 1 to 300 mg or 3 to 300 mg, more preferred between 1 to 250 mg or 5 to 250 mg.

Most preferred is a dose of 5 mg, 15 mg, 60 mg, 120 mg or 240 mg.

It is possible to administer the above defined dosages as a total daily dose either in a single dose daily or divided into sub-doses for administration twice or more times daily.

In a further aspect a dose between 0.001 to 35 mg/kg body weight, between 0.01 to 35 mg/kg body weight, between 0.1 to 25 mg/kg body weight, or between 0.5, 1, 2, 3, 4, 5, 6, 7, 8, 9, 10 and up to 20 mg/kg body weight can be administered. Particularly preferred is a dose of 120 mg for patients with > 50 kg body weight and of 60 mg for patients with < 50 kg body weight, in each case once or twice daily.

In a further aspect it is possible to select one of the above defined dosages as an initial dose and subsequently administer 1 or more times the same or varying doses of those defined above in repeating intervals of 1 to 7 days, 1 to 5 days, preferably of 1 to 3 days, or every second day.

The initial dose and the subsequent doses can be selected among the above defined dosages and adjusted / varied in accordance with the need of the MDS patient within the provided ranges.

In particular, the amount of subsequent doses can be appropriately selected depending on the individual patient, the course of disease and the treatment response. It is possible to administer 1, 2, 3, 4, 5, 6, 7, and more subsequent doses.

It is possible that the initial dose is equal or different to the one or more subsequent doses. It is further possible, that the subsequent doses are equal or different.

The repeating intervals can be of the same length or can be varied depending on the individual patient, the course of disease and the treatment response.

Preferably, the subsequent doses are of decreasing amount with increasing number of subsequent dosing.

Preferably a dose of between 3 mg and 300 mg, more preferred between 5 mg and 250 mg, most preferred of 5 mg, 15 mg, 60 mg, 120 mg or 240 mg is administered once daily over a treatment period of at least 3 days, at least 5 days, at least 7 days. In a further preferred aspect a dose of 60 mg or 120 mg is administered once daily. In a further preferred aspect a total daily dose of 120 mg is administered by administering twice daily a 60 mg dose.

In a further preferred aspect a total daily dose of 240 mg is administered by administering twice daily a 120 mg dose. Said doses turned out to be safe and well tolerated.

The preferred dosing regimen further showed fast oral absorption with detectable levels as early as 15 to 30 minutes post-dose. The absorption level can be maintained stable even upon repeated dosing and no critical accumulation is observed.

The preferred dosing regimen further turned out to efficiently decrease mean serum iron levels and mean calculated transferrin saturation and to shift the mean serum hepcidin peak, indicating its efficiency for treating MDS.

In a further aspect of the invention, the initial and one or more subsequent dosing is adjusted depending on the hemoglobin concentration of the treated patient. The hemoglobin concentration is determined with conventional methods.

### Ferroportin (Fpn) Inhibitor Compounds

The present disclosure relates to the new medical use of the compounds of the formula (I) as defined herein:

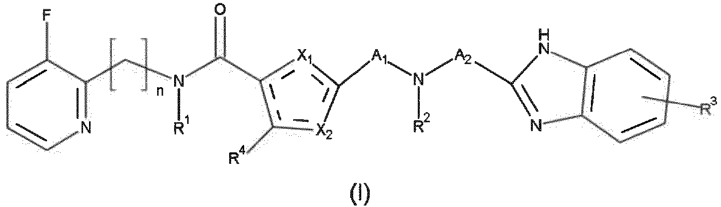

Therein and throughout the disclosure, the substituent groups have the meaning as defined in detail anywhere herein:
Optionally substituted alkyl preferably includes: linear or branched alkyl preferably containing 1 to 8, more preferably 1 to 6, particularly preferably 1 to 4, even more preferred 1, 2 or 3 carbon atoms, also being indicated as C₁-C₄-alkyl or C₁-C₃-alkyl.

Optionally substituted alkyl further includes cycloalkyl containing preferably 3 to 8, more preferably 5 or 6 carbon atoms.

Examples of alkyl residues containing 1 to 8 carbon atoms include: a methyl group, an ethyl group, an n-propyl group, an i-propyl group, an n-butyl group, an i-butyl group, a sec-butyl group, a t-butyl group, an n-pentyl group, an i-pentyl group, a sec-pentyl group, a t-pentyl group, a 2-methylbutyl group, an n-hexyl group, a 1-methylpentyl group, a 2-methylpentyl group, a 3-methylpentyl group, a 4-methylpentyl group, a 1-ethylbutyl group, a 2-ethylbutyl group, a 3-ethylbutyl group, a 1,1-dimethylbutyl group, a 2,2-dimethylbutyl group, a 3,3-dimethylbutyl group, a 1-ethyl-1-methylpropyl group, an n-heptyl group, a 1-methylhexyl group, a 2-methylhexyl group, a 3-methylhexyl group, a 4-methylhexyl group, a 5-methylhexyl group, a 1-ethylpentyl group, a 2-ethylpentyl group, a 3-ethylpentyl group, a 4-ethylpentyl group, a 1,1-dimethylpentyl group, a 2,2-dimethylpentyl group, a 3,3-dimethylpentyl group, a 4,4-dimethylpentyl group, a 1-propylbutyl group, an n-octyl group, a 1-methylheptyl group, a 2-methylheptyl group, a 3-methylheptyl group, a 4-methylheptyl group, a 5-methylheptyl group, a 6-methylheptyl group, a 1-ethylhexyl group, a 2-ethylhexyl group, a 3-ethylhexyl group, a 4-ethylhexyl group, a 5-ethylhexyl group, a 1,1-dimethylhexyl group, a 2,2-dimethylhexyl group, a 3,3-dimethylhexyl group, a 4,4-dimethylhexyl group, a 5,5-dimethylhexyl group, a 1-propylpentyl group, a 2-propylpentyl group, etc. Those containing 1 to 4 carbon atoms (C₁-C₄-alkyl), such as in particular methyl, ethyl, n-propyl, i-propyl, n-butyl, i-butyl, sec-butyl, and t-butyl are preferred. C₁-C₃ alkyl, in particular, methyl, ethyl, propyl and i-propyl are more preferred. Most preferred are C₁ and C₂ alkyl, such as methyl and ethyl.

Cycloalkyl residues containing 3 to 8 carbon atoms preferably include: a cyclopropyl group, a cyclobutyl group, a cyclopentyl group, a cyclohexyl group, a cycloheptyl group and a cyclooctyl group. A cyclopropyl group, a cyclobutyl group, a cyclopentyl group and a cyclohexyl group are preferred. A cyclopropyl group is particularly preferred.

Substituents of the above-defined optionally substituted alkyl preferably include 1, 2 or 3 of the same or different substituents, selected, for example, from the group consisting of: halogen as defined below, such as preferably F, cycloalkyl as defined above, such as preferably cyclopropyl, optionally substituted heteroaryl as defined below, such as preferably a benzimidazolyl group, optionally substituted amino as defined below, such as preferably an amino group or benzyloxycarbonylamino, a carboxyl group, an aminocarbonyl group as defined below, as well as an alkylene group such as in particular a methylene-group, forming for example a methylene-substituted ethyl-group (CH₃-(C=CH₂)- or 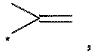 wherein * indicates the binding site).

Within the meaning of the present disclosure, halogen includes fluorine, chlorine, bromine and iodine, preferably fluorine or chlorine, most preferred is fluorine.

Examples of a linear or branched alkyl residue substituted by halogen and containing 1 to 8 carbon atoms include:
a fluoromethyl group, a difluoromethyl group, a trifluoromethyl group, a chloromethyl group, a dichloromethyl group, a trichloromethyl group, a bromomethyl group, a dibromomethyl group, a tribromomethyl group, a 1-fluoroethyl group, a 1-chloroethyl group, a 1-bromoethyl group, a 2-fluoroethyl group, a 2-chloroethyl group, a 2-bromoethyl group, a difluoroethyl group such as a 1,2-difluoroethyl group, a 1,2-dichloroethyl group, a 1,2-dibromoethyl group, a 2,2-difluoroethyl group, a 2,2-dichloroethyl group, a 2,2-dibromoethyl group a 2,2,2-trifluoroethyl group, a heptafluoroethyl group, a 1-fluoropropyl group, a 1-chloropropyl group, a 1-bromopropyl group, a 2-fluoropropyl group, a 2-chloropropyl group, a 2-bromopropyl group, a 3-fluoropropyl group, a 3-chloropropyl group, a 3-bromopropyl group, a 1,2-difluoropropyl group, a 1,2-dichloropropyl group, a 1,2-dibromopropyl group, a 2,3-difluoropropyl group, a 2,3-dichloropropyl group, a 2,3-dibromopropyl group, a 3,3,3-trifluoropropyl group, a 2,2,3,3,3-pentafluoropropyl group, a 2-fluorobutyl group, a 2-chlorobutyl group, a 2-bromobutyl group, a 4-fluorobutyl group, a 4-chlorobutyl group, a 4-bromobutyl group, a 4,4,4-trifluorobutyl group, a 2,2,3,3,4,4,4-heptafluorobutyl group, a perfluorobutyl group, a 2-fluoropentyl group, a 2-chloropentyl group, a 2-bromopentyl group, a 5-fluoropentyl group, a 5-chloropentyl group, a 5-bromopentyl group, a perfluoropentyl group, a 2-fluorohexyl group, a 2-chlorohexyl group, a 2-bromohexyl group, a 6-fluorohexyl group, a 6-chlorohexyl group, a 6-bromohexyl group, a perfluorohexyl group, a 2-fluoroheptyl group, a 2-chloroheptyl group, a 2-bromoheptoyl group, a 7-fluoroheptyl group, a 7-chloroheptyl group, a 7-bromoheptyl group, a perfluoroheptyl group, etc. Fluoroalkyl, difluoroalkyl and trifluoroalkyl are mentioned in particular, and trifluoromethyl and mono- and difluoroethyl is preferred. Particularly preferred is trifluoromethyl.

Examples of a cycloalkyl-substituted alkyl group include the above-mentioned alkyl residues containing 1 to 3, preferably 1 cycloalkyl group such as, for example: cyclopropylmethyl, cyclobutylmethyl, cyclopentylmethyl cyclohexylmethyl, 2-cyclopropylethyl, 2-cyclobutylethyl, 2-cyclopentylethyl 2-cyclohexylethyl, 2- or 3-cyclopropylpropyl, 2- or 3-cyclobutylpropyl, 2- or 3-cyclopentylpropyl, 2- or 3-cyclohexylpropyl, etc. Preferred is cyclopropylmethyl.

Examples of a heteroaryl-substituted alkyl group include the above-mentioned alkyl residues containing 1 to 3, preferably 1 (optionally substituted) heteroaryl group, such as, for example a pyridinyl, a pyridazinyl, a pyrimidinyl, a pyrazinyl, a pyrazolyl, an imidazolyl, a benzimidazolyl, a thiophenyl, or an oxazolyl group, such as pyridine-2-yl-methyl, pyridine-3-yl-methyl, pyridine-4-yl-methyl, 2-pyridine-2-yl-ethyl, 2-pyridine-1-yl-ethyl, 2-pyridine-3-yl-ethyl, pyridazine-3-yl-methyl, pyrimidine-2-yl-methyl, pyrimidine-4-yl-methyl, pyrazine-2-yl-methyl, pyrazol-3-yl-methyl, pyrazol-4-yl-methyl, pyrazol-5-yl-methyl, imidazole-2-yl-methyl, imidazole-5-yl-methyl, benzimidazol-2-yl-methyl, thiophen-2-yl-methyl, thiophen-3-yl-methyl, 1,3-oxazole-2-yl-methyl.

Preferred is an alkyl group which is substituted with a benzimidazolyl group, such as benzimidazol-2-yl-methyl and benzimidazol-2-yl-ethyl.

Examples of an amino-substituted alkyl residue include the above-mentioned alkyl residues containing 1 to 3, preferably 1 (optionally substituted) amino group, as defined below, such as, for example, aminoalkyl (NH₂-alkyl) or mono- or dialkylamino-alkyl, such as aminomethyl, 2-aminoethyl, 2- or 3-aminopropyl, methylaminomethyl, methylaminoethyl, methylaminopropyl, 2-ethylaminomethyl, 3-ethylaminomethyl, 2-ethylaminoethyl, 3-ethylaminoethyl, etc. with 3-aminopropyl being preferred, or an alkyl group, which may be substituted with an optionally substituted alkyloxycarbonylamino group such as a group according to formula 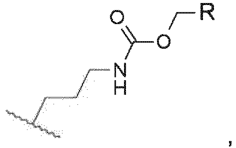 wherein R defines a phenyl group, forming a benzyloxycarbonylaminopropyl group.

Optionally substituted amino according to the invention preferably includes: amino (-NH₂), optionally substituted mono- or dialkylamino (alkyl-NH-, (alkyl)₂N-), wherein with respect to "alkyl" reference can be made to the definition of optionally substituted alkyl above. Preferred is mono- or dimethylamino, mono- or diethylamino and monopropylamino. Most preferred is an amino group (-NH₂), and monopropylamino.

Further, in the sense of the present disclosure, a carboxyl group indicates a group [-(C=O)-OH] and an aminocarbonyl group indicates a group [NH₂-(C=O)-].

Optionally substituted alkoxy includes an optionally substituted alkyl-O-group, wherein reference may be made to the foregoing definition of the alkyl group. Preferred alkoxy groups are linear or branched alkoxy groups containing up to 6 carbon atoms such as a methoxy group, an ethoxy group, an n-propyloxy group, an i-propyloxy group, an n-butyloxy group, an i-butyloxy group, a sec-butyloxy group, a t-butyloxy group, an n-pentyloxy group, an i-pentyloxy group, a sec-pentyloxy group, a t-pentyloxy group, a 2-methylbutoxy group, an n-hexyloxy group, an i-hexyloxy group, a t-hexyloxy group, a sec-hexyloxy group, a 2-methylpentyloxy group, a 3-methylpentyloxy group, a 1-ethylbutyloxy group, a 2-ethylbutyloxy group, a 1,1-dimethylbutyloxy group, a 2,2-dimethylbutyloxy group, a 3,3-dimethylbutyloxy group, a 1-ethyl-1-methylpropyloxy group, as well as cycloalkyloxy groups such as a cyclopentyloxy group or a cyclohexyloxy group. A methoxy group, an ethoxy group, an n-propyloxy group and an i-propyloxy group are preferred. A methoxy and ethoxy group is more preferred. Particularly preferred is a methoxy group.

Throughout the disclosure, optionally substituted alkanediyl is preferably a divalent straight-chained or branched alkanediyl radical having from 1 to 6, preferably from 1 to 4, more preferably 1, 2 or 3 carbon atoms, which can optionally carry from 1 to 3, preferably 1 or 2 substituents selected from the group consisting of halogen, hydroxyl (-OH), an oxo group (C=O; forming a carbonyl or acyl group [-(C=O)-]) and an alkyl group as defined above such as preferably methyl. The following may be mentioned as preferred examples: methylene, ethane-1,2-diyl, ethane-1,1-diyl, propane-1,3-diyl, propane-1,1-diyl, propane-1,2-diyl, propane-2,2-diyl, butane-1,4-diyl, butane-1,2-diyl, butane-1,3-diyl, butane-2,3-diyl, butane-1,1-diyl, butane-2,2-diyl, butane-3,3-diyl, pentane-1,5-diyl, etc. Particularly preferred is methylene, ethane-1,2-diyl, ethane-1,1-diyl, propane-1,3-diyl, propane-2,2-diyl, and butane-2,2-diyl. Most preferred are methylene, ethane-1,2-diyl and propane-1,3-diyl.

A preferred substituted alkanediyl radical is a hydroxy-substituted alkanediyl such as a hydroxy-substituted ethanediyl, an oxo-substituted alkanediyl such as an oxo-substituted methylene or ethanediyl radical, forming a carbonyl or an acyl (acetyl) group, a halogen substituted alkanediyl group such as an alkanediyl group being substituted with one or two halogen atoms selected from F and Cl, preferably 2,2-di-fluoro-ethanediyl, or an alkanediyl group which is substituted with a methyl group.

According to the present disclosure it is further possible that A¹, having the meaning of a linear or branched alkanediyl group as defined above, and R², having the meaning of an optionally substituted alkyl group as defined above, together with the nitrogen atom to which they are bonded form an optionally substituted 4- to 6-membered ring, which may be substituted with 1 to 3 substituents as defined above. Accordingly, A' and R² may together from a group according to one the following formulae 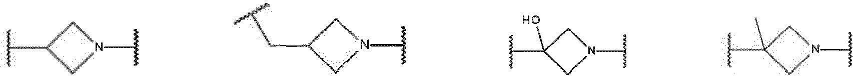 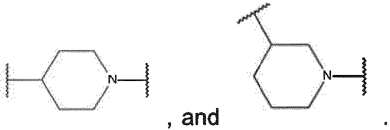 Therein a (substituted or unsubstituted) 4-membered ring-formation is preferred, such as very particularly a group 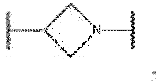 Therein the lefthand binding site indicates the direct binding site to the heterocyclic 5-membered ring between the positions X¹ and X² in formula (I) of the present invention. The right-hand binding site indicates the binding site to the group A² having the meaning of an alkanediyl group as defined herein.

In the formula (I) as defined anywhere herein n has the meaning of an integer of 1 to 3, including 1, 2 or 3 thus indicating a methylene-group, an ethane-1,2-diyl group or a propane-1,3-diyl group. More preferably n is 1 or 2 and even more preferably n is 1, indicating a methylene group.

In the present disclosure the individual substituents of the formula (I) above may have the following meaning:
A) X¹ is N or O; and
   X² is N, S or O;
   with the proviso that X¹ and X² are different;
   thus forming 5-membered heterocycles according to the formulae 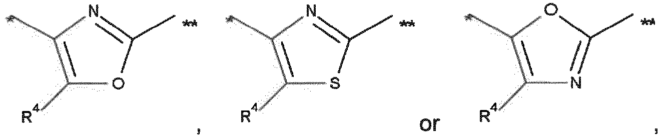
   wherein * indicates the binding site to the aminocarbonyl-group and ** indicates the binding site to the A¹-group.
B) n is an integer of 1, 2 or 3; preferably n is 1 or 2, more preferably n is 1.
C) R' is selected from the group consisting of
   - hydrogen and
   - optionally substituted alkyl (as defined above);
   preferably R' is hydrogen or methyl, more preferably R¹ is hydrogen.
D) R² is selected from the group consisting of
   - hydrogen, and
   - optionally substituted alkyl (as defined above);
   preferably R² is hydrogen or C₁-C₄-alkyl, more preferably R² is hydrogen or methyl, even more preferably R² is hydrogen.
E) R³ indicates 1, 2 or 3 optional substituents, which may independently be selected from the group consisting of
   - halogen (as defined above),
   - cyano,
   - optionally substituted alkyl (as defined above),
   - optionally substituted alkoxy (as defined above), and
   - a carboxyl group (as defined above);

   preferably R³ indicates 1 or 2 optional substituents, which may independently be selected from the consisting of
      - halogen,
      - cyano,
      - alkyl (as defined above), which may be substituted with 1, 2 or 3 halogen atoms (as defined above),
   optionally substituted alkoxy (as defined above), and
   a carboxyl group (as defined above);
   more preferably R³ indicates 1 or 2 optional substituents, which may independently be selected from the group consisting of
      - F and Cl,
      - cyano,
      - trifluoromethyl,
      - methoxy, and
      - a carboxyl group;
   even more preferably R³ is hydrogen, indicating an unsubstituted terminal benzimidazolyl-ring in formula (I).
F) R⁴ is selected from the group consisting of
   - hydrogen,
   - halogen (as defined above),
   - C₁-C₃-alkyl, and
   - halogen substituted alkyl (as defined above); preferably R⁴ is selected from the group consisting of
   - hydrogen
   - Cl,
   - methyl, ethyl, iso-propyl, and
   - trifluoromethyl;

   more preferably R⁴ is selected from the group consisting of
      - hydrogen,
      - Cl,
      - methyl, and
      - trifluoromethyl;
   more preferably R⁴ is selected from the group consisting of
      - hydrogen,
      - Cl, and
      - methyl;
   even more preferably R⁴ is hydrogen.
G) A' is alkanediyl;
   preferably A' is methylene or ethane-1,2-diyl, more preferably A' is ethane-1,2-diyl.
H) A² is alkanediyl;
   preferably A² is methylene, ethane-1,2-diyl or propane-1,3-diyl;
   more preferably A² is methylene or ethane-1,2-diyl;
   even more preferably A² is ethane-1,2-diyl.
I) or A' and R² together with the nitrogen atom to which they are bonded form an optionally substituted 4- to 6-membered ring as defined above;
   therein A¹ and R² together with the nitrogen atom to which they are bonded preferably form an optionally substituted 4-membered ring as defined above;
   therein A' and R² together with the nitrogen atom to which they are bonded more preferably form an unsubstituted 4-membered ring (azetidinyl-ring).

The substituents of the compounds of the following (I) may in particular have the following meaning:
n has any of the meanings according to B) above and the remaining substituents may have any of the meanings as defined in A) and C) to I).

R¹ has any of the meanings according to C) above and the remaining substituents may have any of the meanings as defined in A) and B) and D) to I).

R² has any of the meanings according to D) above and the remaining substituents may have any of the meanings as defined in A) to C) and E) to H) or I).

R³ has any of the meanings according to E) above and the remaining substituents may have any of the meanings as defined in A) to D) and F) to I).

R⁴ has any of the meanings according to F) above and the remaining substituents may have any of the meanings as defined in A) to E) and G) to I).

A' has any of the meanings according to G) above and the remaining substituents may have any of the meanings as defined in A) to F) and H) or I).

A² has any of the meanings according to H) above and the remaining substituents may have any of the meanings as defined in A) to G) and I).

R² and A¹ have any of the meanings as defined in I) and the remaining substituents may have any of the meanings as defined in A) to C), E), F) and H).

In a preferred embodiment of the present disclosure the compounds of general formula (I) are defined by
X¹ is N or O; and
X² is N. S or O;
with the proviso that X¹ and X² are different;
R¹ is hydrogen;
n is 1, 2 or 3;
A' is methylene or ethane-1,2-diyl;
A² is methylene, ethane-1,2-diyl or propane-1,3-diyl;
R² is hydrogen or C₁-C₄-alkyl;
   or
A¹ and R² together with the nitrogen atom to which they are bonded form an optionally substituted 4-membered ring;
R³ indicates 1 or 2 optional substituents, which may independently be selected from the group consisting of
   - halogen,
   - cyano,
   - alkyl, which may be substituted with 1, 2 or 3 halogen atoms,
   - optionally substituted alkoxy, and
   - a carboxyl group;
R⁴ is selected from the group consisting of
   - hydrogen
   - Cl.
   - methyl, ethyl, iso-propyl, and
   - trifluoromethyl.

In a further preferred embodiment the compounds of general formula (I) are defined by
X¹ is N or O: and
X² is N. S or O;
with the proviso that X¹ and X² are different;
R¹ is hydrogen;
n is 1 or 2:
   A' is methylene or ethane-1,2-diyl;
   A² is methylene, ethane-1,2-diyl or propane-1,3-diyl;
   R² is hydrogen or methyl;
   or A' and R² together with the nitrogen atom to which they are bonded form an unsubstituted 4-membered ring;
   R³ indicates 1 or 2 optional substituents, which may independently be selected from the group consisting of
      - F and Cl.
      - cyano,
      - trifluoromethyl,
      - methoxy, and
      - a carboxyl group;
   R⁴ is selected from the group consisting of
      - hydrogen,
      - Cl,
      - methyl, and
      - trifluoromethyl.

In a further preferred embodiment the compounds of general formula (I) are defined by
X' is N or O; and
X² is N, S or O;
with the proviso that X¹ and X² are different;
R¹ is hydrogen;
n is 1;
A¹ is methylene or ethane-1,2-diyl;
A² is methylene, ethane-1,2-diyl or propane-1,3-diyl;
R² is hydrogen;
or A¹ and R² together with the nitrogen atom to which they are bonded form an unsubstituted 4-membered ring;
R³ indicates hydrogen, thus forming an unsubstituted terminal benzimidazolyl-ring;
R⁴ is selected from the group consisting of
   - hydrogen,
   - Cl, and
   - methyl.

In a further preferred embodiment the compounds of general formula (I) are defined by
X¹ is N or O; and
X² is N, S or O;
with the proviso that X¹ and X² are different;
R¹ is hydrogen;
n is 1:
A' is methylene or ethane-1,2-diyl;
A² is methylene, ethane-1,2-diyl or propane-1,3-diyl;
R² is hydrogen;
or A' and R² together with the nitrogen atom to which they are bonded form an unsubstituted 4-membered ring;
R³ indicates hydrogen, thus forming an unsubstituted terminal benzimidazolyl-ring; and
R⁴ is hydrogen.

In a further aspect the present disclosure relates to the new use and method of treatment as defined herein, wherein the compounds according to formula (I), or its salts, solvates, hydrates and polymorphs, are selected from compounds of the formula (I) as shown above, wherein
n = 1;
R³ = hydrogen;
R⁴ = hydrogen;
A' = methylene or ethane-1,2-diyl;
A² = methylene, ethane-1,2-diyl or propane-1,3-diyl;
or A¹ and R² together with the nitrogen atom to which they are bonded form an optionally
substituted 4-membered ring,
forming compounds according to formula (II) or (III):

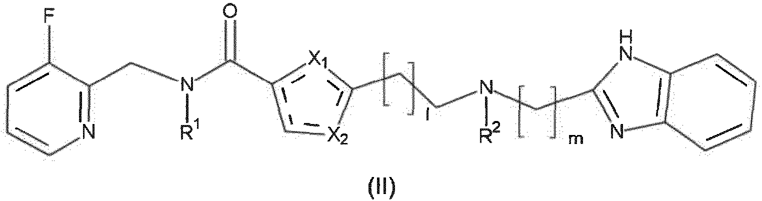

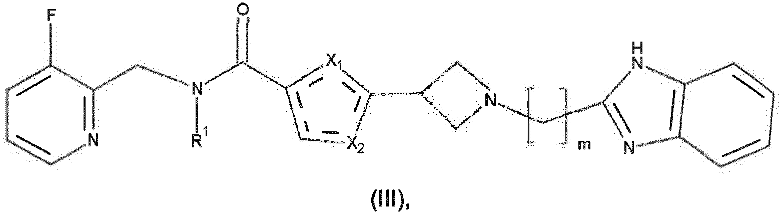

wherein in formula (II) and/or (III)
I is 0 or 1:
   m is an integer of 1, 2 or 3 and
   X¹, X², R¹ and R² have the meaning as defined for compounds of formula (I) anywhere herein.

Preferably, in the formulae (II) and (III) X¹ and X² have the meaning as defined above in A).

In formula (II) R¹ and R² are preferably hydrogen.

In formula (III) R¹ is preferably hydrogen and m is preferably 2.

In a further preferred embodiment the compounds of genera formula (II) are defined by
X' and X² are selected from N and O and are different;
R¹ = hydrogen;
R² = hydrogen;
I = 1: and
m = 2.

In a further preferred aspect, the present disclosure relates to the new use and method of treatment as defined herein, wherein the compounds according to formula (I) are used in the form of its pharmaceutically acceptable salts, or solvates, hydrates and polymorphs thereof.

With respect to suitable pharmaceutically acceptable salts of the compounds of the formulae (I), (II) and (III) as defined anywhere herein reference is made to the international applications WO2017/068089, WO2017/068090 and in particular WO2018/192973. Further compounds acting as ferroportin inhibitors and being suitable in the treatment of MDS as defined herein are those as described in WO2020/123850 A1, . Particular compounds among those described in WO2020/123850 A1 being suitable in the treatment of MDS as defined herein can be selected from the group consisting of:

| **Structure** | **IUPAC Name** | **Mass Found (M+1)** |
|---|---|---|
| 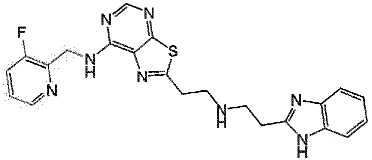 | 2-(2-{[2-(1H-1 ,3-benzodiazol-2-yl)ethyl]amino}ethyl)-N-[(3-fluoropyridin-2-yl)methyl]-[1,3]thiazolo[5,4-d]pyrimidin-7-amine | 449.2 |
| 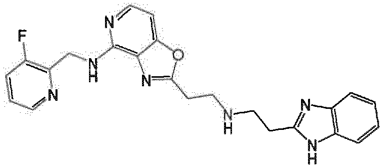 | 2-(2-{[2-(1H-1,3- benzodiazol-2-yl)ethyl]amino}ethyl)-N-[(3-fluoropyridin-2-yl)methyl]-[1,3]oxazolo[4,5-c]pyridin-4-amine | 432.2 |
| 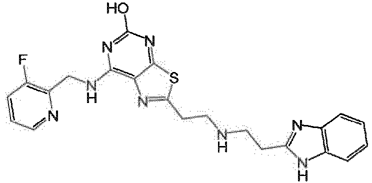 | 2-(2-{[2-(1H-1,3- benzodiazol-2-yl)ethyl]amino}ethyl)-7-{[(3-fluoropyridin-2-yl)methyl]amino }-[1,3]thiazolo[5,4-d]pyrimidin-5-ol | 465.1 |
| 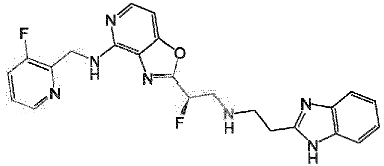 | 2-[(1R)-2-{[2-(1H-1,3-benzodiazol-2-yl)ethyl]amino}-1-fluoroethyl]-N-[(3-fluoropyridin-2-yl)methyl]-[1,3]oxazolo[4,5-c]pyridin-4-amine | 450.2 |
| 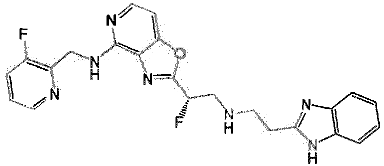 | 2-[(1S)-2-{[2-(1H-1,3-benzodiazol-2-yl]ethyl]amino}-1-fluoroethyl]-N-[(3-fluoropyridin-2-yl)methyl]-[1,3]oxazolo[4,5-c]pyridin-4-amine | 450.2 |
| 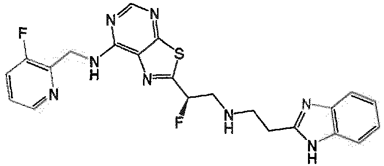 | 2-[(1R)-2-{[2-(1H-1,3-benzodiazol-2-yl)ethyl]amino)-1-fluoroethyl]-N-[(3-fluoropyridin-2-yl)methyl]-[1,3]thiazolo[5,4-d]pyrimidin-7-amine | 467 |

In a further preferred aspect the present disclosure relates to the use and method of treatment as defined herein, wherein the pharmaceutically acceptable salts of the compounds of the formulae (I), (II) or (III) or of the compounds according to WO2020/123850 A1 are selected from salts with acids from the group consisting of benzoic acid, citric acid, fumaric acid, hydrochloric acid, lactic acid, malic acid, maleic acid, methanesulfonic acid, phosphoric acid, succinic acid, sulfuric acid, tartaric acid and toluenesulfonic acid. Preferably acids from the group consisting of citric acid, hydrochloric acid, maleic acid, phosphoric acid and sulfuric acid are selected.

In a further preferred aspect the present disclosure relates to the new use and method of treatment as defined herein, wherein the pharmaceutically acceptable salts of the compounds of the formulae (I), (II) or (III) are selected from mono-salts (1:1 salts), triple salts (1:3 salts) and salts being characterized by a ratio of compound (I), (II) or (III) to acid of 1-2 : 1-3; including solvates, hydrates and polymorphs thereof.

Therein, the salts of the compounds (I), (II) or (III) may be characterized by a selected ratio of base : acid, i.e. compound (I), (II) or (III) : the acids as defined above, in the range of 1.0 to 2.0 (mol base) : 1.0 to 3.0 (mol acid). In a particular embodiment the selected ratio of base : acid is 1.0 to 2.0 (mol base) : 1.0 to 2.0 (mol acid).

Particular examples comprise the following ratios of base : acid, i.e. compound (I), (II) or (III) : the acids as defined above:
1.0 (mol base) : 1.0 (mol acid);
1.0 (mol base) : 1.25 (mol acid):
1.0 (mol base) : 1.35 (mol acid);
1.0 (mol base) : 1.5 (mol acid);
1.0 (mol base) : 1.75 (mol acid);
1.0 (mol base) : 2.0 (mol acid);
1.0 (mol base) : 3.0 (mol acid); and
2.0 (mol base) : 1.0 (mol acid).

Therein, a salt having a ratio of base : acid of 1 : 1 is also called "mono-salt(s)" or "1 : 1 salt(s)". For example, a mono-HCl salt is also designated as 1HCl or 1HCl salt.

Therein, a salt having a ratio of base : acid of 1 : 2 is also called "di-salt(s)" or "1 : 2 salt(s)". For example, a di-HCl salt is also designated as 2HCl or 2HCl salt.

Therein, a salt having a ratio of base : acid of 1 : 3 is also called "tri-salt(s)", "triple salts(s)" or "1 : 3 salt(s)". For example, a tri-HCl salt is also designated as 3HCl or 3HCl salt.

A salt having a ratio of base : acid of 1 : 1.25 is also called "1 : 1.25 salt(s)".

A salt having a ratio of base : acid of 1 : 1.35 is also called "1 : 1.35 salt(s)".

A salt having a ratio of base : acid of 1 : 1.5 is also called "1 : 1.5 salt(s)".

A salt having a ratio of base : acid of 1 : 1.75 is also called "1 : 1.75 salt(s)".

A salt having a ratio of base : acid of 2 : 1 is also called "hemi-salt(s)" or "2 : 1 salt(s)".

The salts of the compounds of formulae (I), (II) or (III) according to the present disclosure may be present in amorphous, polymorphous, crystalline and/or semi-crystalline (partly crystalline) form as well as in the form of a solvate of the salt. Preferably salts of the compounds of formulae (I), (II) or (III) according to the present invention are present in crystalline and/or semi-crystalline (partly crystalline) form and/or in the form of solvates thereof.

The preferable crystallinity of the salts or salt solvates can be determined by using conventional analytical methods, such as especially by using the various X-ray methods, which permit a clear and simple analysis of the salt compounds. In particular, the grade of crystallinity can be determined or confirmed by using Powder X-ray diffraction (reflection) methods or by using Powder X-ray diffraction (transmission) methods (PXRD). For crystalline solids having identical chemical composition, the different resulting crystal gratings are summarized by the term polymorphism. Regarding solvates, hydrates and polymorphs and salts with particular crystallinity reference is made to the international application WO2018/192973.

In a further preferred aspect the present disclosure relates to the use and method of treatment as defined herein, wherein the compounds of the formulae (I), (II) or (III) are selected from the group consisting of:

| **Exp No.** | **Structure** | **Exp No.** | **Structure** |
|---|---|---|---|
| 1 | 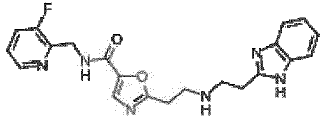 | 126 | 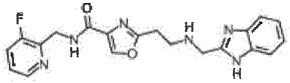 |
| 2 | 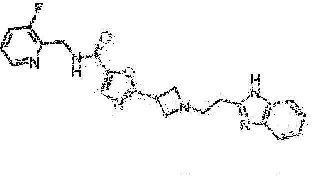 | 127 | 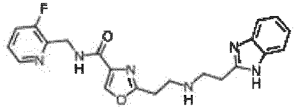 |
| 4 | 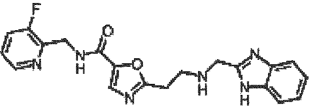 | 193 | 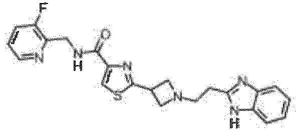 |
| 40 | 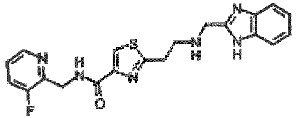 | 206 | 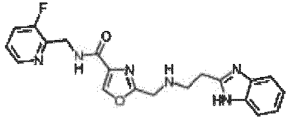 |
| 94 | 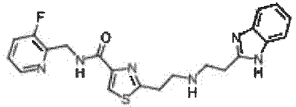 | 208 | 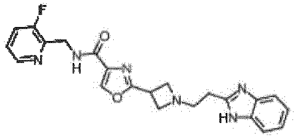 |
| 118 | 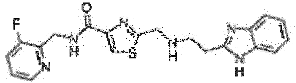 | 233 | 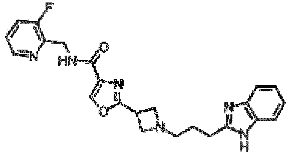 |

and its pharmaceutically acceptable salts, solvates, hydrates and polymorphs.

In a further preferred aspect the present disclosure relates to the new use and method of treatment as defined herein, wherein the compounds of the formulae (I), (II) or (III) are selected from the group consisting of:

| **Exp. No.** | **Structure** | **Exp. No.** | **Structure** |
|---|---|---|---|
| 1 | 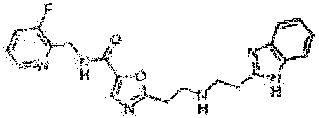 | 127 | 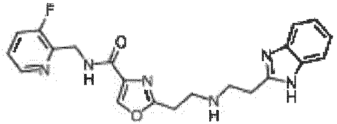 |
| 40 | 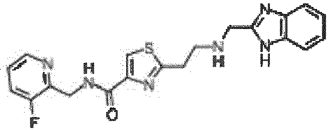 | 208 | 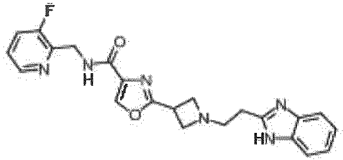 |
| 94 | 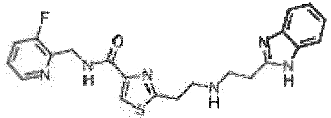 | | |

and its pharmaceutically acceptable salts, solvates, hydrates and polymorphs.

In a further preferred aspect the present disclosure relates to the new use and method of treatment as defined herein, wherein the compounds of the formulae (I), (II) or (III) are selected from the group consisting of:

| **Exp. No.** | **Structure** | **Exp. No.** | **Structure** |
|---|---|---|---|
| 1 | 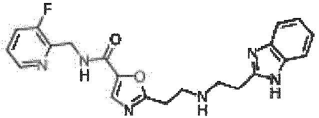 | 127 | 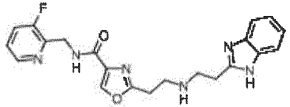 |
| 2 | 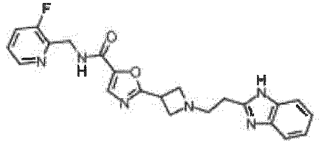 | 206 | 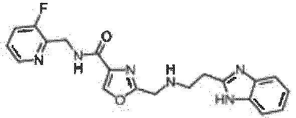 |
| 4 | 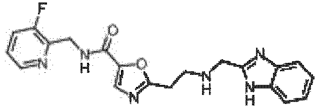 | 208 | 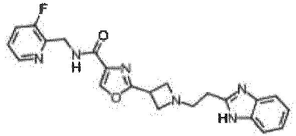 |
| 126 | 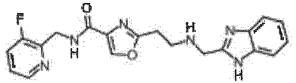 | 233 | 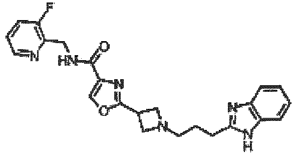 |

In a further preferred aspect the present disclosure relates to the new use and method of treatment as defined herein, wherein the compounds of the formulae (I), (II) or (III) are selected from the group consisting of:

| **Exp. No.** | **Structure** | **Exp. No.** | **Structure** |
|---|---|---|---|
| 1 | 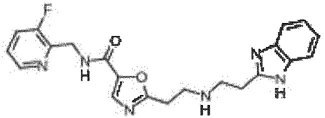 | 127 | 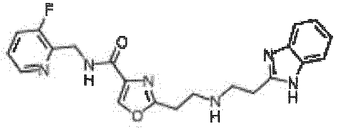 |
| 208 | 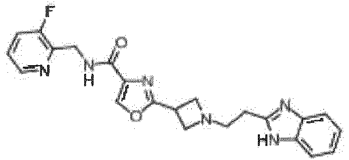 | | |

and its pharmaceutically acceptable salts, solvates, hydrates and polymorphs.

In an even more preferred aspect of the disclosure the compounds of the formulae (I), (II) or (III) are selected from the group consisting of: 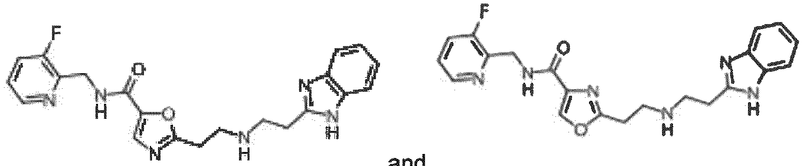 and its pharmaceutically acceptable salts, solvates, hydrates and polymorphs.

Note that only compound 127 forms part of the present invention.

In a further preferred aspect of the invention the compounds of the formulae (I), (II) or (III) are selected from the group consisting of the following salts:
a 1:1 sulfate salt having the formula 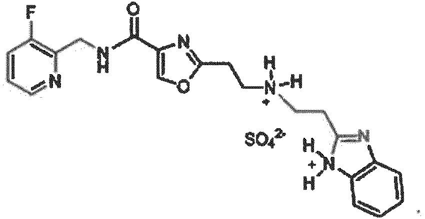
a 1:1 phosphate salt having the formula 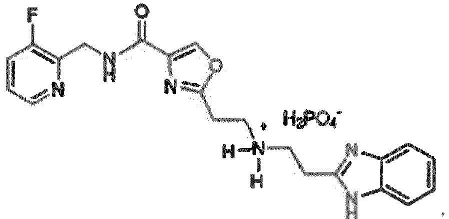
a 2 : 1 phosphate salt (hemiphosphate) 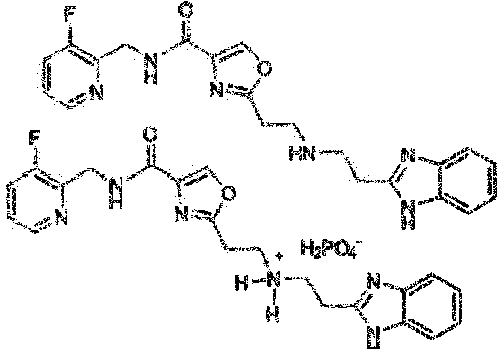
a 1:3 HCl salt having the formula 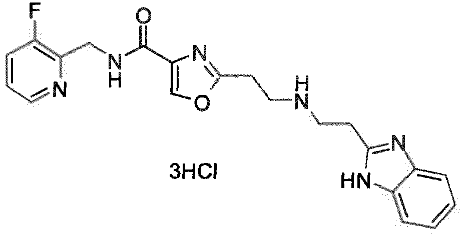
and polymorphs thereof.

As described in WO2017/068089, WO2017/068090 and WO2018/192973 the compounds of the formula (I) act as ferroportin inhibitors. Regarding the ferroportin inhibitor activity of the compounds reference is thus made to said international applications.

### Medicaments containing the Ferroportin Inhibitor Compounds

A further aspect of the disclosure relates to a medicament or a pharmaceutical composition containing one or more of the compounds of the formulae (I), (II) or (III) as defined anywhere herein for the new use and method of treatment of MDS as defined anywhere herein.

Such medicament may further contain one or more pharmaceutical carriers and/or one or more auxiliaries and/or one or more solvents.

Preferably, the medicament is in the form of an oral dosage form, e.g. such as defined above.

Preferably the pharmaceutical carriers and/or auxiliaries and/or solvents are selected among suitable compounds for preparing oral dosage forms.

The said pharmaceutical compositions contain, for example up to 99 weight-% or up to 90 weight-% or up to 80 weight-% or or up to 70 weight-% of the ferroportin inhibitor compounds of the present invention, the remainder being each formed by pharmacologically acceptable carriers and/or auxiliaries and/or solvents and/or optionally further pharmaceutically active compounds.

Therein, the pharmaceutically acceptable carriers, auxiliary substances or solvents are common pharmaceutical carriers, auxiliary substances or solvents, including various organic or inorganic carrier and/or auxiliary materials as they are customarily used for pharmaceutical purposes, in particular for solid medicament formulations. Examples include excipients, such as saccharose, starch, mannitol, sorbitol, lactose, glucose, cellulose, talcum, calcium phosphate, calcium carbonate; binding agents, such as cellulose, methylcellulose, hydroxypropylcellulose, polypropyl pyrrolidone, gelatine, gum arabic, polyethylene glycol, saccharose, starch; disintegrating agents, such as starch, hydrolyzed starch, carboxymethylcellulose, calcium salt of carboxymethylcellulose, hydroxypropyl starch, sodium glycol starch, sodium bicarbonate, calcium phosphate, calcium citrate; lubricants, such as magnesium stearate, talcum, sodium laurylsulfate; flavorants, such as citric acid, menthol, glycin, orange powder; preserving agents, such as sodium benzoate, sodium bisulfite, paraben (for example methylparaben, ethylparaben, propylparaben, butylparaben); stabilizers, such as citric acid, sodium citrate, acetic acid and multicarboxylic acids from the titriplex series, such as, for example, diethylenetriaminepentaacetic acid (DTPA); suspending agents, such as methycellulose, polyvinyl pyrrolidone, aluminum stearate; dispersing agents; diluting agents, such as water, organic solvents; waxes, fats and oils, such as beeswax, cocoa butter; polyethylene glycol; white petrolatum; etc.

Liquid medicament formulations, such as solutions, suspensions and gels usually contain liquid carrier, such as water and/or pharmaceutically acceptable organic solvents. Furthermore, such liquid formulations can also contain pH-adjusting agents, emulsifiers or dispersing agents, buffering agents, preserving agents, wetting agents, gelatinizing agents (for example methylcellulose), dyes and/or flavouring agents, for example as defined above. The compositions may be isotonic, that is, they can have the same osmotic pressure as blood. The isotonicity of the composition can be adjusted by using sodium chloride and other pharmaceutically acceptable agents, such as, for example, dextrose, maltose, boric acid, sodium tartrate, propylene glycol and other inorganic or organic soluble substances. The viscosity of the liquid compositions can be adjusted by means of a pharmaceutically acceptable thickening agent, such as methylcellulose. Other suitable thickening agents include, for example, xanthan gum, carboxymethylcellulose, hydroxypropylcellulose, carbomer and the like. The preferred concentration of the thickening agent will depend on the agent selected.

Pharmaceutically acceptable preserving agents can be used in order to increase the storage life of the liquid composition. Benzyl alcohol can be suitable, even though a plurality of preserving agents including, for example, paraben, thimerosal, chlorobutanol and benzalkonium chloride can also be used.

### Combination Therapy

A further object of the present invention relates to medicaments or combined preparations containing one or more of the ferroportin inhibitor as defined in claim 1 and at least one further pharmaceutically active compound ("combination therapy compound"), preferably an additional active compound being useful in the treatment of MDS as defined herein. Preferred combination therapy compounds are in particular compounds used in the prophylaxis and treatment of ineffective erythropoiesis, including erythropoietin-stimulating agents, erythropoietin (EPO), and antibiotics as well as immunosuppressive agents. Known drugs used in the treatment of MDS include lenalidomide, antithymocyte globulin, and azacitidine. Chemotherapy followed by a stem-cell transplant from a donor is a further treatment option for MDS patients. Further preferred combination therapy compounds are selected from medicaments for treating iron overload and the associated symptoms. Most preferred combination therapy compounds are iron-chelating compounds, or compounds for the prophylaxis and treatment of any of the states, disorders or diseases accompanying or resulting from iron overload and MDS. Suitable combination therapy drug compounds (co-drugs) may be selected from pharmaceutically active compounds for the prophylaxis and treatment of MDS and the associated symptoms. In particular, co-drugs for treating ineffective hematopoiesis, in particular ineffective erythropoiesis, such as erythropoietin stimulating agents or erythropoietin are preferred. In a further embodiment, the at least one additional pharmaceutically active combination therapy compound is selected from drugs for reducing iron overload (e.g. Tmprss6-ASO) and iron chelators, in particular curcumin, SSP-004184, Deferitrin, deferasirox, deferoxamine and deferiprone as well as hydroxyurea or with JAK2 inhibitors.

Further preferred combination therapy compounds may be selected from drugs for treating MDS, such as lenalidomide, antithymocyte globulin, and azacytidine or antibiotics as well as immunosuppressive agents.

Further possible co-drugs include erythroid maturating agents, such as Luspatercept, or other erythroid maturation agents / erythroid stimulating agents, such as e.g. EPO, Epoetin, or Darbepoetin, or synthetic human hepcidin (LJPC-401), the hepcidin peptidomimetic PTG-300 and the anti-sense oligonucleotide targeting Tmprss6 (IONIS-TMPRSS6-L RX).

In a further aspect the present invention relates to the use and medical treatment of MDS as defined herein, wherein the ferroportin inhibitor compound as defined herein is administered to the patient in need thereof in a combination therapy with one or more of the combination therapy compounds (co-drugs) defined above in a fixed dose or free dose combination for sequential use. Such a combination therapy comprises co-administration of the ferroportin inhibitor as defined in the present invention with the at least one additional pharmaceutically active compound (drug/combination therapy compound).

Combination therapy in a fixed dose combination therapy comprises co-administration of the ferroportin inhibitor compounds as defined herein with the at least one additional pharmaceutically active compound in a fixed-dose formulation.

Combination therapy in a free dose combination therapy comprises co-administration of the ferroportin inhibitor compounds as defined herein and the at least one additional pharmaceutically active compound in free doses of the respective compounds, either by simultaneous administration of the individual compounds or by sequential use of the individual compounds distributed over a time period.

In a preferred embodiment, a combination therapy comprises concurrent administration of the oral ferroportin inhibitor according to Example compound No. 127, described herein, and erythropoietin.

In a further embodiment, a combination therapy comprises concurrent administration of the oral ferroportin inhibitor according to Example compound No. 127, described herein, and Luspatercept.

In a further embodiment, a combination therapy comprises concurrent administration of the oral ferroportin inhibitor according to Example compound No. 127, described herein, and the iron chelator deferasirox.

A further embodiment of the present disclosure relates to a combination therapy as described herein, wherein the ferroportin inhibitor compound is one selected among those described in WO2020/123850 A1, in particular one of the particular example compounds thereof as described above. Preferably, such combination therapy comprises concurrent administration of the ferroportin inhibitor compound and the iron chelator deferasirox.

### DESCRIPTION OF THE FIGURES

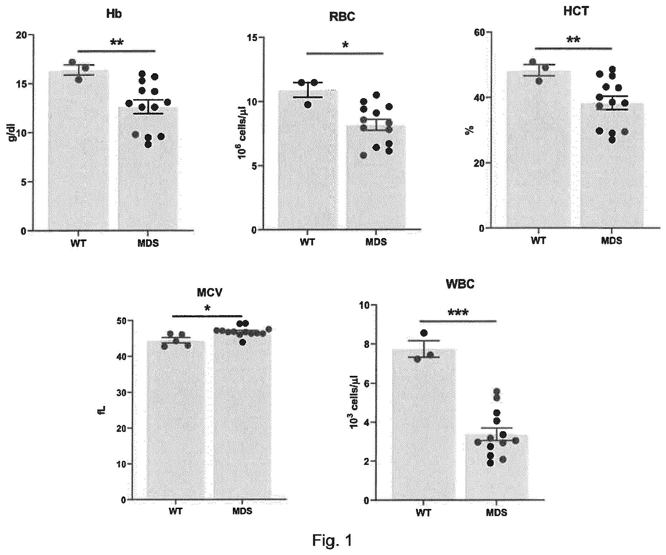

- Figure 1:: Anemia in 3 month-old MDS mice. Blood parameters (hemoglobin, red blood cell number, hematocrit, mean cellular volume and white blood cell count) in 3 month-old wild-type (WT) control mice and myelodysplastic (MDS) mice.

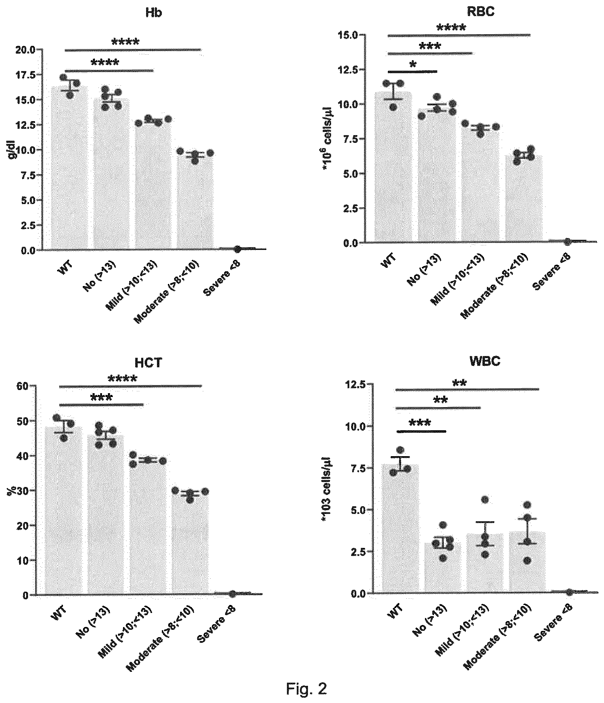

- Figure 2:: MDS mice show very mild, mild and moderate anemia at 3 months of age. Blood parameters (hemoglobin, red blood cell number, hematocrit and white blood cell count) in 3 month-old wild-type (WT) control mice and myelodysplastic (MDS) mice according to the anemia levels (no/very mild anemia: Hb>13 g/dl; mild anemia: 10 g/dl<Hb<13 g/dl; moderate anemia: 8 g/dl<Hb<10 g/dl; severe anemia: Hb<8 g/dl).

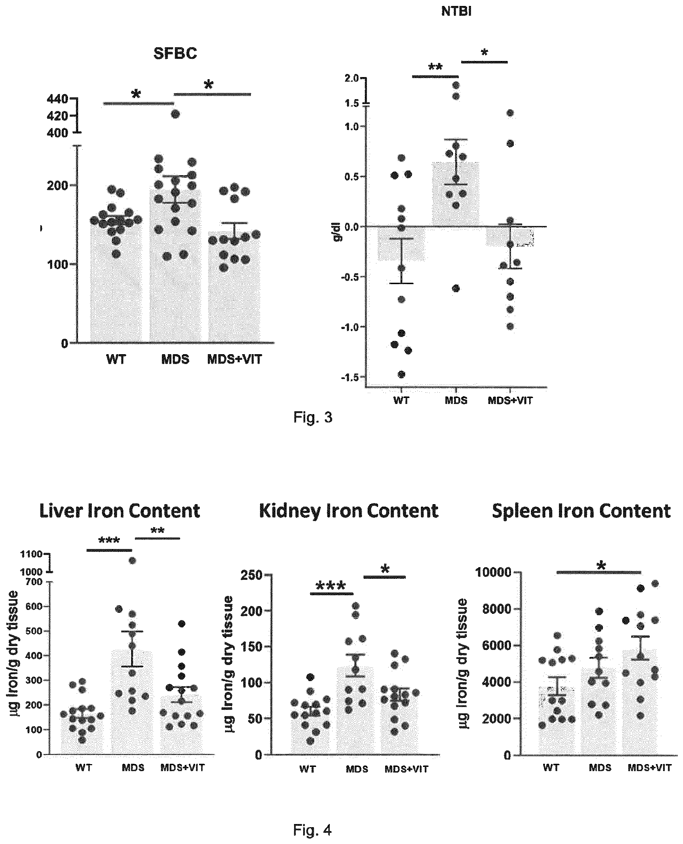

- Figure 3:: Fpn127 treatment reduces serum iron levels and NTBI formation in MDS mice. Total iron (SFBC) and non-transferrin bound iron (NTBI) measurement in the sera of 6 month-old wild-type (WT) control mice and myelodysplastic (MDS) mice untreated or treated with 0.5 mg/ml Fpn127 (MDS+VIT) for 3 months.
- Figure 4:: Fpn127 treatment prevents iron loading in MDS mice. Liver, kidney and spleen iron content in 6 month-old wild-type (WT) control mice and myelodysplastic (MDS) mice untreated or treated with 0.5 mg/ml Fpn127 (MDS+VIT) for 3 months.

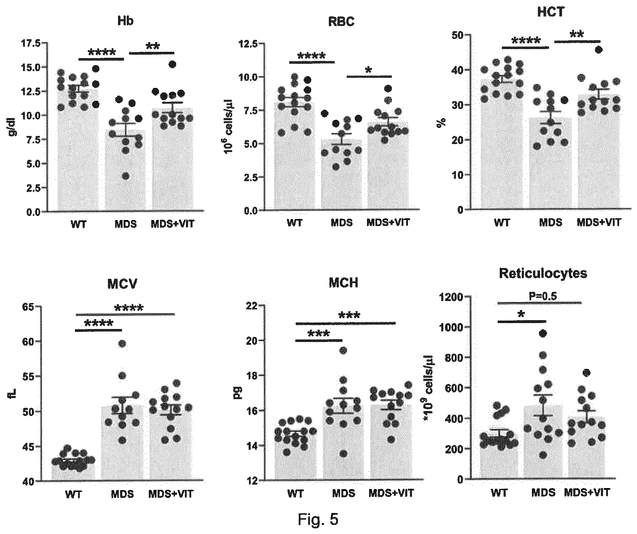

- Figure 5:: Fpn127 treatment improves anemia in MDS mice. Red blood cell parameters (hemoglobin, red blood cell number, hematocrit, mean cellular volume, mean cellular hemoglobin and reticulocyte count) in 6 month-old wild-type (WT) control mice and myelodysplastic (MDS) mice untreated or treated with 0.5 mg/ml Fpn127 (MDS+VIT) for 3 months.

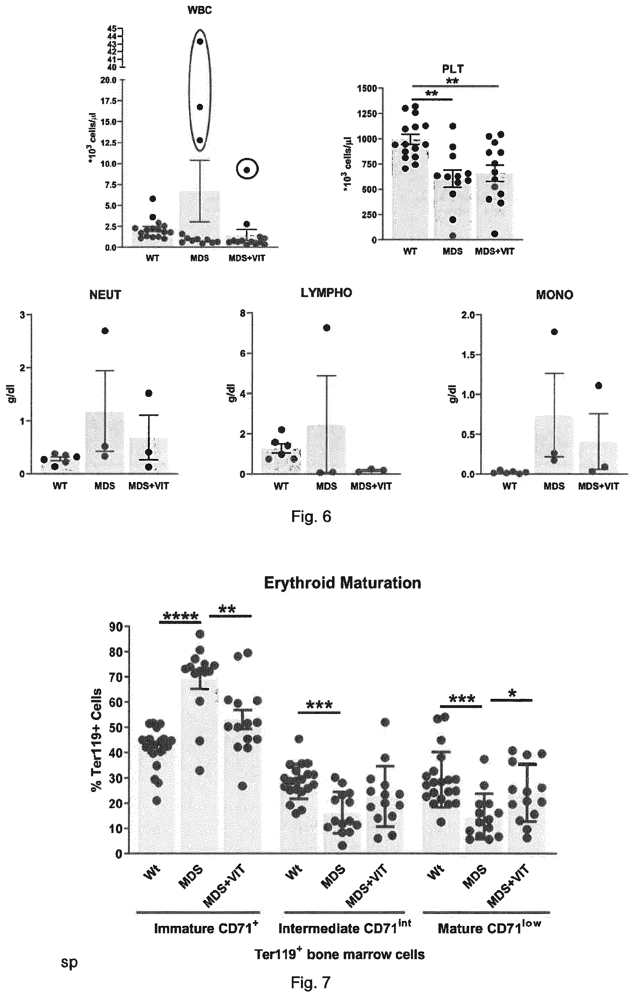

- Figure 6:: Fpn127 treatment shows a trend in reducing progression to leukemia in MDS mice. White blood cell parameters (white blood cell, platelet, neutrophil, lymphocyte and monocyte counts) in 6 month-old wild-type (WT) control mice and myelodysplastic (MDS) mice untreated or treated with 0.5 mg/ml Fpn127 (MDS+VIT) for 3 months.
- Figure 7:: Fpn127 treatment improves bone marrow erythroid maturation in MDS mice. Erythroid immature to mature populations were monitored by progressive loss of CD71 expression on bone marrow Ter119+ erythroid cells in 6 month-old wild-type *(WT)* control mice and myelodysplastic (MDS) mice untreated or treated with 0.5 mg/ml Fpn127 (MDS+VIT) for 3 months.

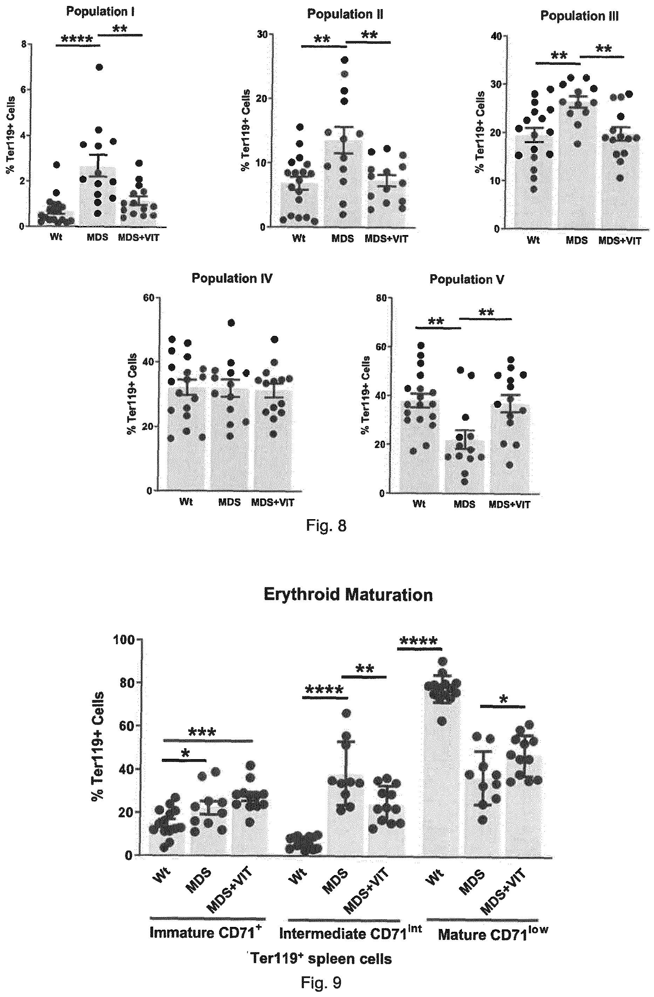

- Figure 8:: Fpn127 treatment improves bone marrow erythroid maturation in MDS mice. Erythroid maturation was evaluated by assessing erythroid populations I to V (I: pro-erythroblasts; II: basophilic erythroblasts; III: polychromatic erythroblasts; IV: orthochromatic erythroblasts/reticulocytes; V: erythrocytes) through progressive loss of CD44 expression on bone marrow Ter119+ erythroid cells in 6 month-old wild-type (WT) control mice and myelodysplastic (MDS) mice untreated or treated with 0.5 mg/ml Fpn127 (MDS+VIT) for 3 months.
- Figure 9:: Fpn127 treatment improves splenic erythroid maturation in MDS mice. Erythroid immature to mature populations were monitored by progressive loss of CD71 expression on splenic Ter119+ erythroid cells in 6 month-old wild-type (WT) control mice and myelodysplastic (MDS) mice untreated or treated with 0.5 mg/ml Fpn127 (MDS+VIT) for 3 months.

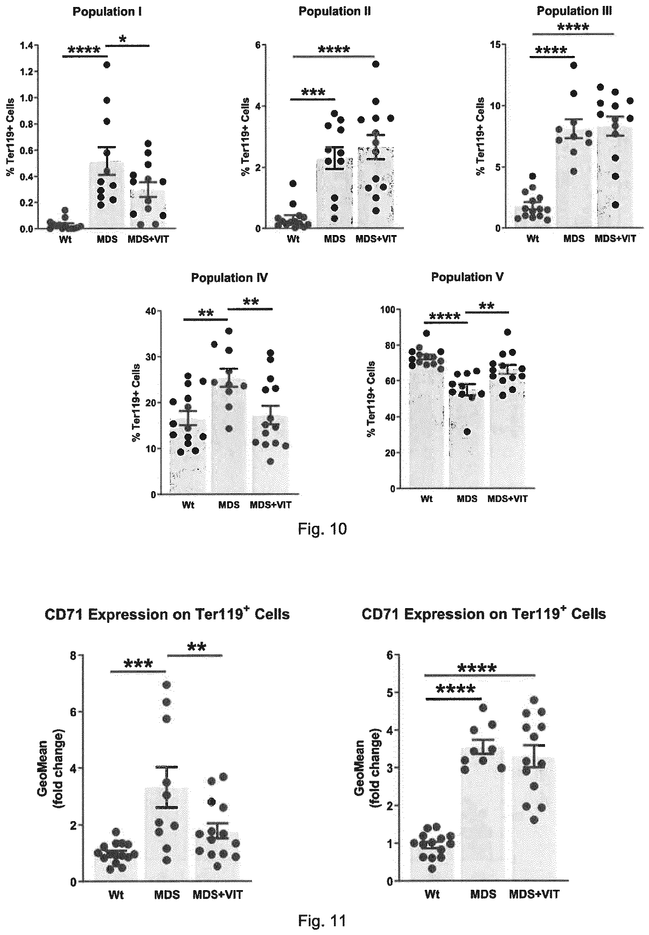

- Figure 10:: Fpn127 treatment improves splenic erythroid maturation in MDS mice. Erythroid maturation was evaluated by assessing erythroid populations I to V (I: pro-erythroblasts; II: basophilic erythroblasts; III: polychromatic erythroblasts; IV: orthochromatic erythroblasts/reticulocytes; V: erythrocytes) through progressive loss of CD44 expression on splenic Ter119+ erythroid cells in 6 month-old wild-type (WT) control mice and myelodysplastic (MDS) mice untreated or treated with 0.5 mg/ml Fpn127 (MDS+VIT) for 3 months.
- Figure 11:: Fpn127 treatment improves erythroid maturation in MDS mice. Improvement of erythroid maturation by Fpn127 was confirmed by monitoring loss of CD71 expression on bone marrow and splenic Ter119+ erythroid cells in 6 month-old wild-type (WT) control mice and myelodysplastic (MDS) mice untreated or treated with 0.5 mg/ml Fpn127 (MDS+VIT) for 3 months.

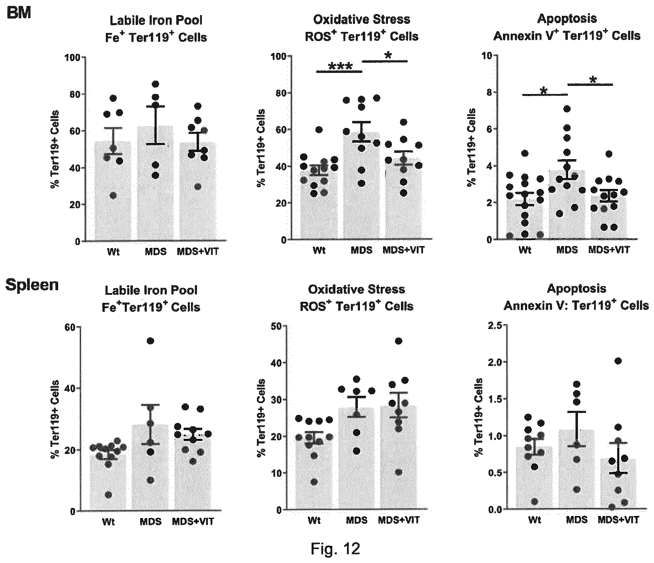

- Figure 12:: Fpn127 treatment ameliorates anemia by decreasing oxidative stress and apoptosis of erythroid precursors in MDS mice. Iron accumulation (labile iron), oxidative stress (ROS) and apoptosis (Annexin V) were monitored by flow cytometry in bone marrow and splenic Ter119+ erythroid cells of 6 month-old wild-type (WT) control mice and myelodysplastic (MDS) mice untreated or treated with 0.5 mg/ml Fpn127 (MDS+VIT) for 3 months.

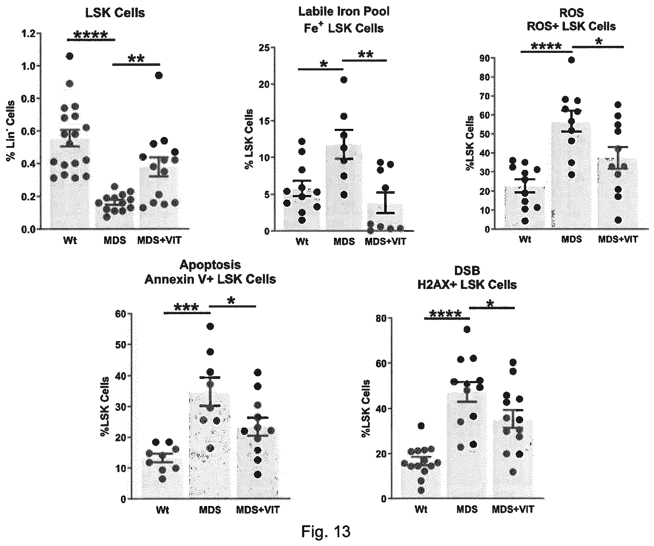

- Figure 13:: Fpn127 treatment improves the overall status of hematopoietic LSK cell in MDS mice. Cell percentage, iron accumulation (labile iron), oxidative stress (ROS) and apoptosis (Annexin V) and double strand break (γH2AX) were monitored by flow cytometry in bone marrow hematopoietic Lin- Sca-1+ ckit+ (LSK) cells of 6 month-old wild-type (WT) control mice and myelodysplastic (MDS) mice untreated or treated with 0.5 mg/ml Fpn127 (MDS+VIT) for 3 months.

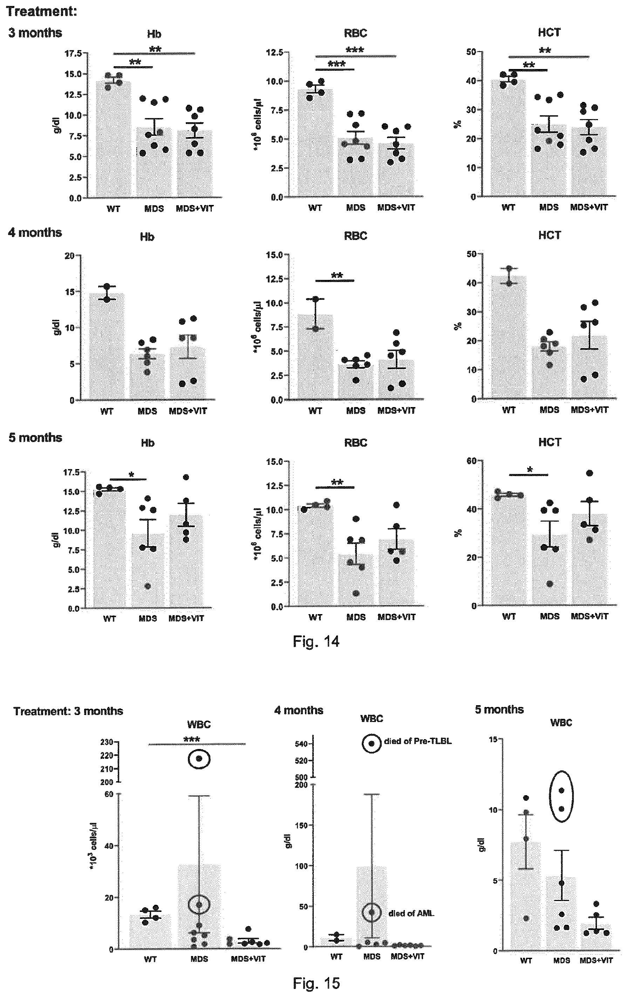

- Figure 14:: Fpn127 treatment improves anemia in older MDS mice. Red blood cell parameters (hemoglobin, red blood cell number, hematocrit) in 8 to 10 month-old wild-type (WT) control mice and myelodysplastic (MDS) mice untreated or treated with 0.5 mg/ml Fpn127 (MDS+VIT) for 3 to 5 months
- Figure 15:: Fpn127 treatment reduces leukemia-related death in older MDS mice. WBC count in 8 to 10 month-old wild-type (WT) control mice and myelodysplastic (MDS) mice untreated or treated with 0.5 mg/ml Fpn127 (MDS+VIT) for 3 to 5 months. As indicated, 2 untreated MDS mice died of leukemia, whereas 2 Fpn127-treated MDS mice died of MDS, as also suggested by the low WBC count.

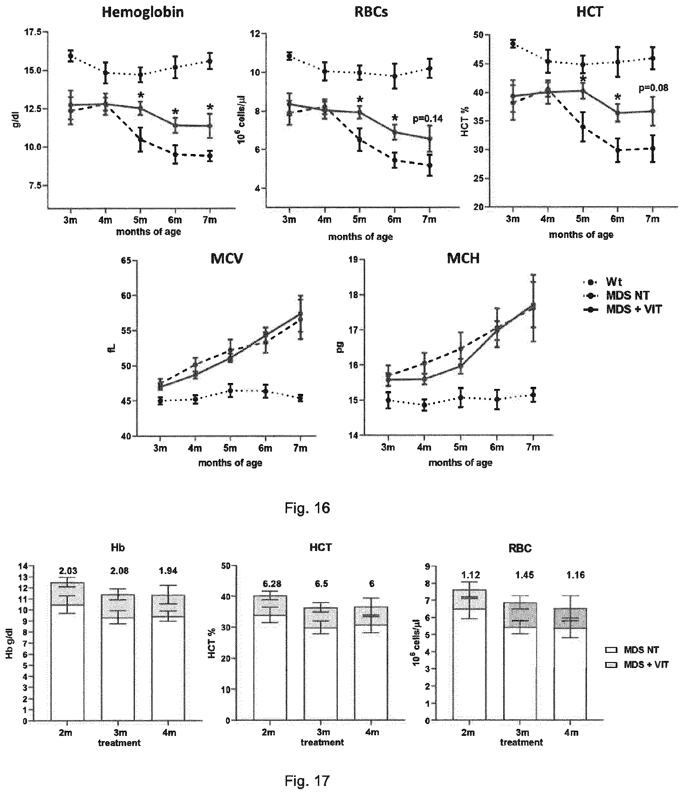

- Figure 16:: VIT-2763 treatment improves anemia in MDS mice. Longitudinal monitoring of blood parameters (hemoglobin - Hb, hematocrit - HCT, red blood cells - RBC) in wild-type (WT) control mice and myelodysplastic (MDS) mice untreated or treated with 0.5 mg/ml VIT-2763 (MDS+VIT) from 3 months of age.
- Figure 17:: VIT-2763 treatment improves anemia in MDS mice. Improvement (Δ, delta) of Hb, HCT and RBC in myelodysplastic (MDS) mice untreated or treated with 0.5 mg/ml VIT-2763 (MDS+VIT) starting at 3 months of age. Δ is shown after 2, 3 and 4 months of treatment (5, 6 and 7 months of age).

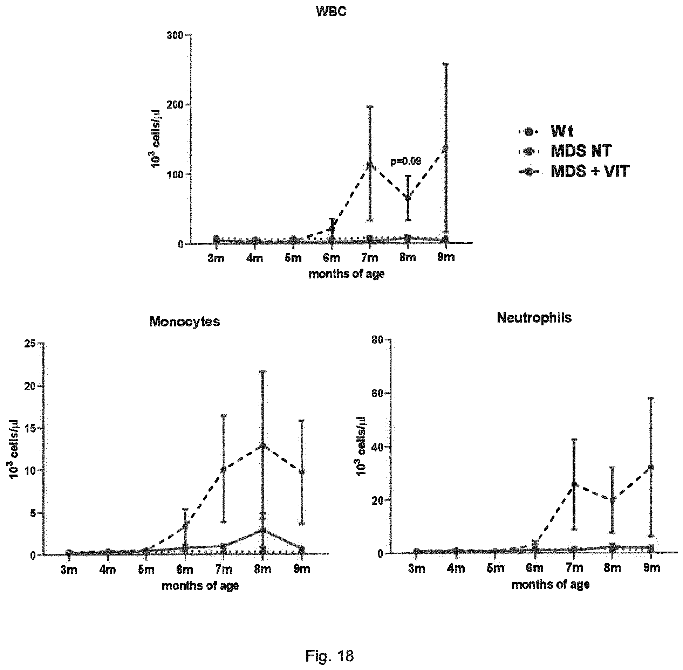

- Figure 18:: VIT-2763 treatment delays leukemia evolution in MDS mice. Longitudinal monitoring of total white blood cells, monocytes and neutrophils in wild-type (WT) control mice and myelodysplastic (MDS) mice untreated or treated with 0.5 mg/ml VIT-2763 (MDS+VIT) from 3 months of age.

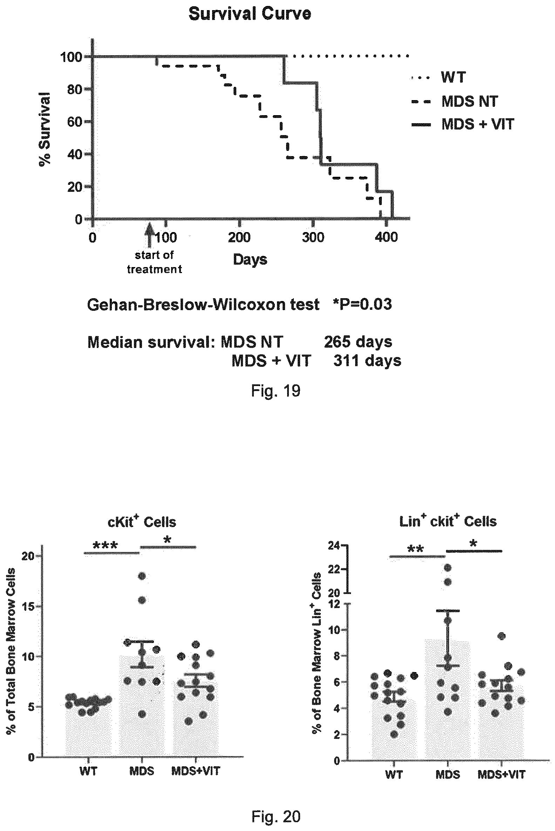

- Figure 19:: VIT-2763 treatment improves MDS mice survival. Kaplan-Meier curve in wild-type (WT) control mice and myelodysplastic (MDS) mice untreated or treated with 0.5 mg/ml VIT-2763 (MDS+VIT) from 3 months of age.
- Figure 20:: VIT-2763 treatment reduces bone marrow immature cells in MDS mice. Percentage of cKit⁺ and Lin⁺ cKit⁺ cells in the bone marrow of wild-type (WT) control mice and myelodysplastic (MDS) mice untreated or treated with 0.5 mg/ml VIT-2763 (MDS+VIT) from 3 to 6 months of age. Immature blasts are within the Lin⁺ cKit⁺ population.

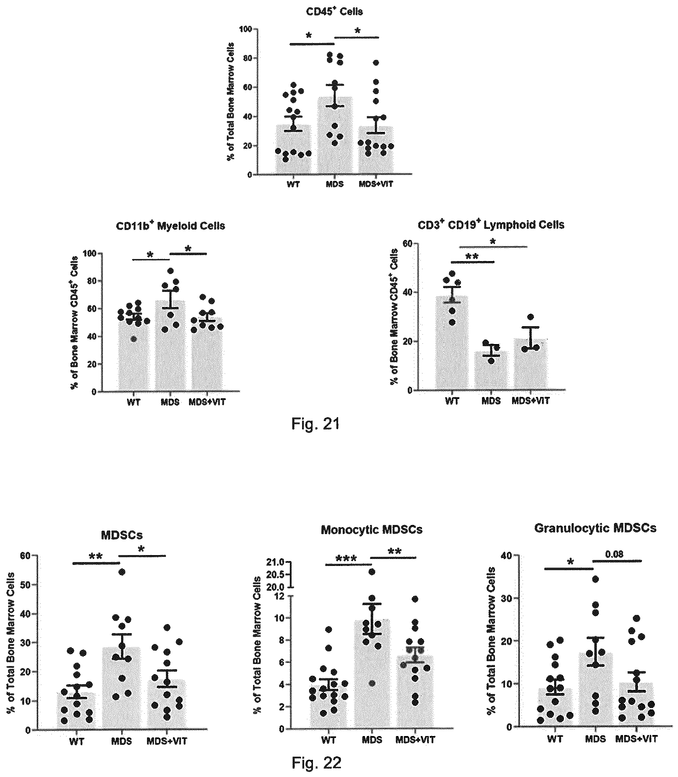

- Figure 21:: VIT-2763 treatment reduces myeloid expansion in the bone marrow of MDS mice. Percentage of CD45+ immune cells, CD11b⁺ myeloid cells and CD3⁺ CD19⁺ lymphoid cells in the bone marrow of wild-type (WT) control mice and myelodysplastic (MDS) mice untreated or treated with 0.5 mg/ml VIT-2763 (MDS+VIT) from 3 to 6 months of age.
- Figure 22:: VIT-2763 treatment reduces myeloid expansion in the bone marrow of MDS mice. Percentage of total CD11b+ Ly6C+ Ly6G+ myeloid-derived suppressor cells (MDSCs), CD11b⁺ Ly6C⁺ monocytic and CD11b⁺ Ly6G⁺ granulocytic MDSCs in the bone marrow of wild-type (WT) control mice and myelodysplastic (MDS) mice untreated or treated with 0.5 mg/ml VIT-2763 (MDS+VIT) from 3 to 6 months of age.

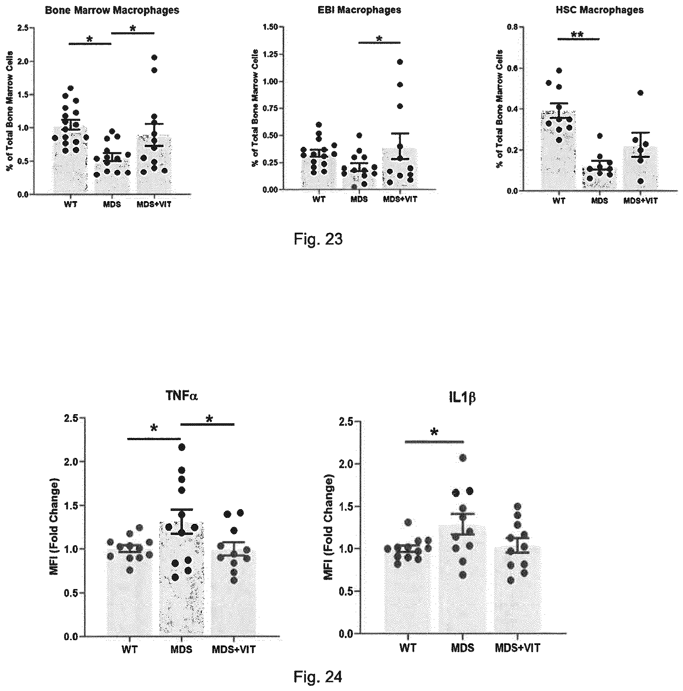

- Figure 23:: VIT-2763 treatment improves macrophage number in the bone marrow of MDS mice. Percentage of total macrophages, erythroblastic island and HSC macrophages in the bone marrow of wild-type (WT) control mice and myelodysplastic (MDS) mice untreated or treated with 0.5 mg/ml VIT-2763 (MDS+VIT) from 3 to 6 months of age.
- Figure 24:: VIT-2763 treatment limits bone marrow macrophage-mediated inflammation in MDS mice. TNFa and IL-1b production in total bone marrow macrophages of wild-type (WT) control mice and myelodysplastic (MDS) mice untreated or treated with 0.5 mg/ml VIT-2763 (MDS+VIT) from 3 to 6 months of age.

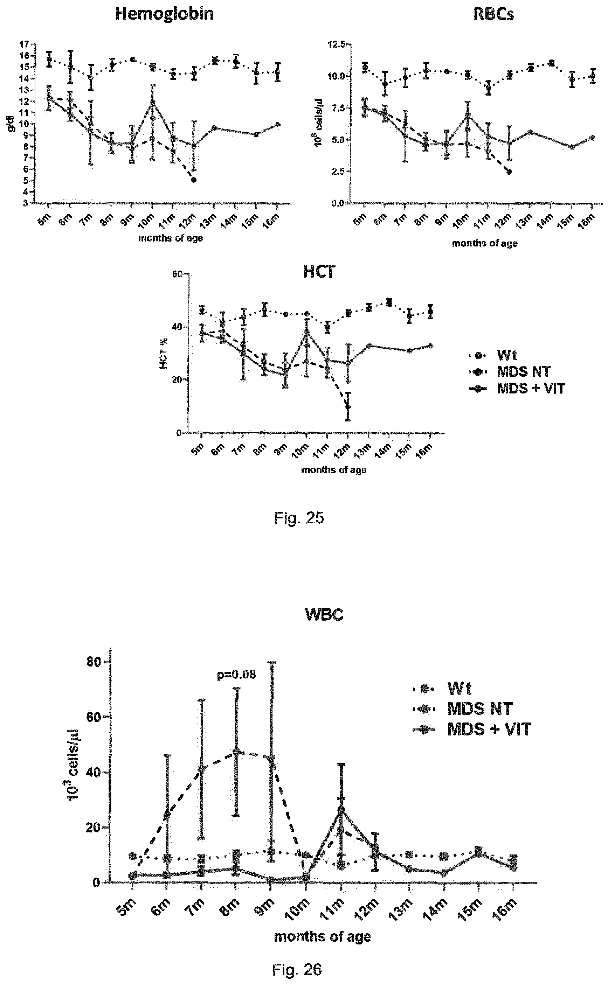

- Figure 25:: VIT-2763 treatment improves anemia in MDS mice. Longitudinal monitoring of blood parameters (hemoglobin - Hb, hematocrit - HCT, red blood cells - RBC) in wild-type (WT) control mice and myelodysplastic (MDS) mice untreated or treated with 0.5 mg/ml VIT-2763 (MDS+VIT) from 5 months of age.
- Figure 26:: VIT-2763 treatment delays leukemia evolution in MDS mice. Longitudinal monitoring of white blood cells in wild-type (WT) control mice and myelodysplastic (MDS) mice untreated or treated with 0.5 mg/ml VIT-2763 (MDS+VIT) from 5 months of age.

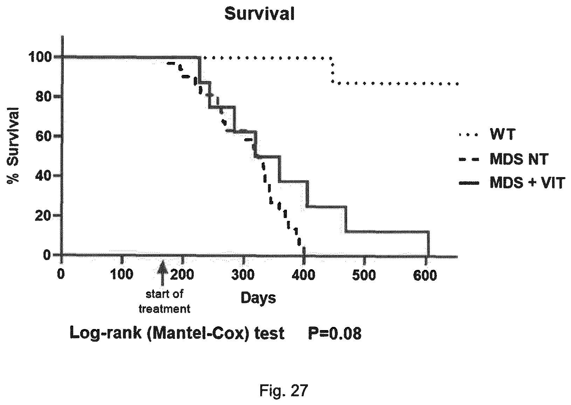

- Figure 27:: VIT-2763 treatment improves MDS mice survival. Kaplan-Meier curve in wild-type (WT) control mice and myelodysplastic (MDS) mice untreated or treated with 0.5 mg/ml VIT-2763 (MDS+VIT) from 5 months of age.

In the Figures "VIT-2763" or "VIT" indicates the test compound Fpn127 (Example Compound No. 127).

### EXAMPLES

The invention is illustrated in more detail by the following examples.

### I. Ferroportin Inhibitor Example Compounds

Regarding the preparation of the specific Ferroportin Inhibitor Example Compounds Nos. 1, 2, 4, 40, 94, 118, 126, 127, 193, 206, 208 and 233 as described herein and the preparation of pharmaceutically acceptable salts thereof reference is made to the international applications WO2017/068089, WO2017/068090 and WO2018/192973.

Regarding the preparation of the specific Ferroportin Inhibitor Compounds described in WO2020/123850 A1 reference is made to the preparation methods described in said international application WO2020/123850 A1.

### II. Pharmacological Assays

### 11.1 Introduction

The orally bioavailable ferroportin inhibitors, such as the clinical stage compound according to Example Compound No. 127 (Fpn127) has been shown to improve ineffective erythropoiesis, ameliorate anemia and prevent NTBI formation and liver iron loading in a mouse model of MDS. Ferroportin inhibitors, such as the clinical stage Example Compound No. 127 further limit iron availability and reactive oxygen species (ROS) in erythroid precursors and thereby prevent their apoptosis and improve the ineffective erythropoiesis. As a result, more RBCs with extended life-span ameliorate anemia and improve tissue oxygenation.

Based on this, the inventors of the present invention have found that the described ferroportin inhibitors are particularly efficient in treating MDS, in particular ineffective erythropoiesis. Patients with MDS, suffering from ineffective erythropoiesis, have reduced Hb levels, which are usually treated with blood transfusions (BT), leading to severe iron overload. In a further aspect, prevention of intestinal iron absorption by ferroportin inhibitors during the intervals between transfusions helps to reduce further iron loading in MDS patients. Further, non-transferrin bound iron (NTBI) is released by macrophages recycling damaged RBCs and triggers oxidative stress and vascular damage. MDS patients have been found to have elevated NTBI levels, this applies to transfused and non-transfused MDS patients.

It has now been found that oral ferroportin inhibitors according to the present invention, such as the ferroportin inhibitor Example Compound No. 127, have the potential to prevent these noxious effects by sequestrating iron in macrophages. With the beneficial effects to be achieved with the ferroportin inhibition therapy on hemoglobin levels, NTBI levels and LPI levels in MDS patients the ferroportin inhibitor compounds of the present invention have the potential to improve the hematological values in MDS patients and in a further aspect may achieve a reduction in transfused RBC units and thus a reduction in transfusion burden for MDS patients.

### II.2 Evaluation of the Ferroportin Inhibitor Compound Fpn127 in a preclinical MDS mouse model

### Background Summary

Patients with myelodysplastic syndromes (MDS) are prone to develop iron overload due to ineffective erythropoiesis, which promotes increased iron absorption, and to chronic transfusions, which are often essential to recover anemia in this patient population. The effect of the ferroportin inhibitor compounds according to the present invention in transfusion-independent and -dependent MDS is evaluated with the Example Compound Fpn127, with the aim to show that iron restriction limits iron absorption, alleviates non-transferrin-bound iron (NTBI) formation and tissue iron deposition and mediates its redistribution and to show whether the ferroportin inhibitors of the present invention are beneficial for reducing overall iron burden by lowering body iron influx consequent to erythropoiesis-driven hepcidin suppression in transfusion-independent MDS. Reduced iron levels and NTBI are considered to have an impact on MDS by alleviating iron-driven cell toxicities (cell death, ROS production) and organ damage, improving bone marrow functionality with positive effects for the microenvironment and erythropoiesis, limiting oxidative damage to hematopoietic stem cells (HSC). In transfusion-dependent MDS, besides reducing iron absorption, FPN inhibition is considered to provide a strategy to restrict RBC-derived iron, leading to its redistribution from iron-sensitive tissues to recycling macrophages. This is considered to lower NTBI levels and therefore exert beneficial effects in transfusion dependent MDS, especially by reducing bone marrow exposure to NTBI. Further, the combination of the ferroportin inhibitors according to the present invention administered in a combination therapy with iron chelation is considered to provide a novel and more effective strategy for body iron removal in transfusion dependent MDS conditions.

### Determination of the Effect of Iron Restriction Induced by the Administration of Fpn127 in a mouse model of MDS at steady-state

The ferroportin inhibitor compound Fpn127 was tested in NUP98-HOXD13 MDS mice. Fpn127 was administered in drinking water containing 1% glucose at the concentration of 0.5 mg/ml.

### Preventive effect:

To study the protective effect of ferroportin inhibitors according to the invention on the progressive development of iron overload and related toxicities, overall 15 3-month-old MDS mice were treated with the Example Compound Fpn127 and compared to age- and sex-matched 15 untreated MDS mice and wild-type control (total of 3 repeated experiments). Mice were treated for 3 months - from 3 to 6 months of age. During the treatment period 2 untreated and 1 treated MDS mice were lost.

MDS mice of 3 months of age already show a certain degree of anemia, which supports to use the ferroportin inhibitors in the treatment starting from 3 months to improve erythropoiesis and anemia (Figure 1).

About 60-70% of MDS mice show a mild to moderate anemia at 3 months of age. According to Hb levels, 3 month-old MDS mice can be divided into 3 groups presenting very mild, mild and moderate anemia (no/very mild anemia: Hb>13 g/dl; mild anemia: 10 g/dl<Hb<13 g/dl; moderate anemia: 8 g/dl<Hb<10 g/dl) (Figure 2). Rarely MDS mice show severe anemia at 3 months age (severe anemia: Hb<8 g/dl). This reflects the situation of MDS patients whose 70-80% present anemia at diagnosis with different degree of severity.

In MDS mice treated for 3 months with Fpn127 from 3 to 6 months of age, the following parameters were monitored:
- Serum iron levels and NTBI;
- Liver iron accumulation;
- Anemia and blood parameters;
- Erythropoiesis - including RBC maturation, erythroid precursor apoptosis and ROS;
- Hematopoietic stem cells - including apoptosis, ROS and DNA damage;
- Bone marrow macrophages and myeloid-derived suppressor cells (not shown).

The Figures 3 to 15 show the results obtained as average of 3 independent experiments.

### Serum iron levels and NTBI:

Serum iron and NTBI levels are elevated in MDS mice compared to control mice and significantly decreased by Fpn127 treatment (Figure 3).

### Liver iron accumulation:

Liver and kidney iron content is elevated in MDS mice compared to controls and significantly decreased by Fpn127 treatment (both in males and females) (Figure 4). By contrast, spleen iron, which is slightly but not significantly elevated in MDS mice due to enhanced iron absorption and modest erythroid expansion, is further elevated upon VIT treatment, in line with VIT-mediated FPN inhibition and splenic macrophage iron accumulation (Figure 4).

### Anemia and blood parameters:

Hb, RBC and HCT are reduced in MDS mice compared to control animals and significantly improved by Fpn127 treatment. Reticulocytes show a trend to improvement after Fpn127 treatment. MCV and MCH remain unchanged (Figure 5).

### White blood cell parameters:

WBC count is reduced in MDS mice. Only MDS mice that develop forms of leukemia show a significant elevation of WBC count - either of the lymphoid or myeloid lineage. While 3 mice show elevated WBC count (one developed leukemia) in the untreated arm, only one mouse show a trend to elevated WBC count in the Fpn127 treatment arm. Reticulocytes show a trend to improvement after Fpn127 treatment. Platelet count is reduced in MDS mice and remains unchanged after Fpn127 treatment (Figure 6).

### Erythropoiesis:

Erythropoiesis is significantly impaired both in the bone marrow and in the spleen of MDS mice compared to controls and significantly improved by Fpn127 treatment. According to the assessment both via CD71 loss and CD44 loss on Ter119 + cells, erythroid cell maturation is ameliorated by iron restriction, resulting in the reduction of cell percentage in the immature populations and increase in that of mature populations (Figures 7 to 10). Overall, this indicates improved RBC maturation and reduced ineffective erythropoiesis (both in the bone marrow and spleen). This is confirmed by a significant reduction in the expression of CD71 on Ter119 + erythroid precursors, especially in the bone marrow (Figure 11).

### Erythropoiesis:

Improved anemia after Fpn127 treatment is associated with reduced ROS formation and apoptosis in Ter119+ erythroid precursors, suggesting that limited oxidative stress and improved cell survival contribute mechanistically to the amelioration of erythropoiesis (Figure 12).

### Hematopoietic stem cells:

Iron restriction by Fpn127 treatment is associated with an overall improvement of the status of hematopoietic LSK stem cells (Figure 13). The pool of LSK cells is reduced in MDS mice and likely preserved by Fpn127 treatment. LSK cells of Fpn127-treated MDS mice show reduced iron accumulation, decreased ROS production and improved cell survival (lower apoptosis), suggesting that the alteration of these events better preserve the HSC pool. In addition, LSK cells show reduced double strand breaks (DSB; lower γH2AX) in Fpn127-treated MDS mice compared to untreated animals. DSBs likely contribute to leukemia progression through the accumulation of mutations in HSPCs which acquire increased propensity to proliferation and clonality.

### Rescuing effect:

To study the rescuing effect of the ferroportin inhibitors according to the invention on the established iron overload and related toxicities in MDS, overall 8 5-month-old MDS mice were treated with the Example Compound Fpn127 and compared to 8 age- and sex-matched MDS untreated mice and wild-type control (1 experiment). Mice were treated from 5 months of age onwards. During the treatment period 3 untreated and 3 treated MDS mice were lost. In this cohort of mice it was failed to observe improved anemia after 3 months of treatment (5 to 8 months of age). However, results from mice at 9 and 10 months of age suggest that a subset of mice can benefit of Fpn127 treatment, with a partial improvement of anemia (Figure 14). Mice died of different causes - 2 untreated MDS mice died of AML and TLL; 2 Fpn127-treated MDS mice died of MDS, with no apparent progression to leukemia (Figure 15). Other 2 mice died without the opportunity to monitor parameters. No molecular analysis of 5 month-old treated mice was obtained as this cohort was kept for further observing and monitoring. To better follow individual blood parameter modulation two cohorts of mice whose treatment has been started at 3 and 5 months of age respectively, are analyzed longitudinally once a month along the treatment.

### Additional Results:

Similar as in the MDS mouse model described above, the kinetic effects of VIT-2763, administered into the drinking water (0.5 mg/ml) was investigated in NUP98-HOXD13 model of MDS.

### Preventive effect - Additional Results:

To study the protective effect of the compound on the progressive development of iron overload and related toxicities, 3-month-old MDS mice were treated and compared to age- and sex-matched untreated MDS mice and wild-type control. Mice were treated with VIT-2763 from 3 months of age on to follow longitudinally blood parameters and survival (Figures 16-19), or for 3 months - from 3 to 6 months of age, for cellular and biochemical analyses (Figures 20-24).

### Anemia and blood parameters - Additional Results:

Hb, HCT and RBC are reduced in MDS mice compared to control animals and significantly improved by VIT-2763 treatment from 5 through 7 months of age (Figure 16). VIT treatment improved Hb levels of about 2 g/dl in MDS mice (Figure 17).

### White blood cell parameters - Additional Results:

WBC count was initially reduced in MDS mice. Only MDS mice that developed leukemia show a significant elevation of WBC count. Interestingly, several MDS mice in the untreated arm showed elevated WBC count and developed leukemia, whereas in the VIT-2763 treatment arm such WBC increase in the peripheral blood is not observed or delayed (Figure 18).

Consistent with a reduced/delayed trend to leukemia development, immature Lin⁺ cKit⁺ cells which contains myeloblasts were increased in MDS mice and reduced following 3 months-long VIT treatment (Figure 20).

Myeloid expansion was reduced in MDS mice after VIT treatment as suggested by the reduced percentage of bone marrow CD11b+ myeloid cells as well as monocytic and granulocytic myeloid-derived suppressor cells (MDSCs) (Figures 21 and 22).

Bone marrow macrophages were significantly reduced in MDS mice. This could be a consequence of poor myeloid terminal differentiation and contribute to ineffective erythropoiesis and HSC loss. VIT treatment improved macrophage number in the bone marrow of MDS mice (Figure 23). Together with this, the production of inflammatory cytokines as TNFα and IL-1β by macrophages was reduced by VIT treatment (Figure 24), with potential beneficial effect on erythropoiesis, HSPCs and the bone marrow microenvironment.

### Rescuing effect - Additional Results:

To study the rescuing effect of the compound on the established iron overload and related toxicities in MDS, 5-month-old MDS mice were treated and compared to age- and sex-matched untreated MDS mice and wild-type controls. Mice were treated from 5 months of age onwards. In this cohort of mice improved anemia was observed after 5 months of treatment (10 months of age). The anemia of MDS mice tends to worsen from 5 months of age on. The results suggest that while in the beginning mice scarcely benefit from the treatment, a subset of mice can benefit at about 10 months of age with a partial improvement of anemia (Figure 25).

Interestingly, MDS mice treated with VIT showed delayed leukemia development (Figure 26), similarly to what was observed in mice treated from 3 months of age on.

While most MDS mice were lost at about 12/13 months of age, two MDS mice treated with VIT over a total of 10, were still alive at 15 months of age, with stable blood parameters (Hb about 9 g/dl) and one of them reached 20 months of age (Figure 25).

Overall, VIT treatment in older MDS mice translated into a modest median survival improvement of about 16 days compared to untreated mice (Figure 27). However, this clearly demonstrated that a subset of mice can specifically benefit from VIT treatment, with untreated MDS animals all dying within 400 days of life, and those MDS benefiting from VIT treatment reaching up to 600 days.

### III. Transfusion Burden

Transfusion burden in a subject treated according to the methods of the present invention can be evaluated by determining the transfusion requirement of the patient, e.g. via the required amount and/or frequency of red blood cell transfusion by conventional and clinically acknowledged assessment.

### IV. Iron Levels

Iron levels, such as, e.g., liver, kidney or myocardial iron levels can be determined using conventional assay(s). For example, iron levels (e.g., liver iron concentration, kidney iron concentration or myocardial iron concentration) can be determined by magnetic resonance imaging.

### V. Serum Ferritin Level Determination

Serum ferritin levels can be determined using conventional assay(s).

### VI. Erythroid Response

The duration of the erythroid response can be calculated for a subject who achieves a response using the following algorithm:
First Day of Response = the first day of the first 12-week interval showing response. Last Day of Response = last day of the last consecutive 129-week interval showing response. Date of Last Assessment = either the last visit date for subjects still on drug or the date of discontinuation for subjects who discontinued from the treatment.

The duration of the erythroid response can be calculated as follows, depending on whether or not the response ends before the Date of Last Assessment:
1. a subject whose response does not continue to the end of a treatment period, the duration of response is not censored, and is calculated as:$Response Duration = Last Day of Response - First Day of Response + 1 ;$
2. a subject who continues to exhibit an erythroid response at the end of a treatment period, the end date of the response is censored and duration of the response is calculated as:$Response Duration = Date of Last Response Assessment - First Day of Response + 1 .$

The time to the first erythroid response can be calculated as follows:
the day from the first dose of study drug to the First Day of Response starts will be calculated using:$Time to Response = First Day of Response - Date of First Study Drug + 1 .$

### VII. Hemo-globin Determination

Hemoglobin levels can be determined using conventional assay(s).

### VIII. Quality of Life

The assessment of quality of life can be evaluated using the Short Form (36) Health Survey (SF-26) and/or the Functional Assessment of Cancer Therapy-Anemia (FACT-An) as described e.g. in WO2016/183280 can be used.

### IX. Efficacy of the ferroportin inhibitor VIT-2653 (Example Compound No. 40) to attenuate plasma iron, oxidant stress and renal injury following red blood cell transfusion in guinea pigs

The efficacy of the ferroportin inhibitor compounds of the present invention in the treatment of MDS is further supported by the results of J. H. Baek et al. "Ferroportin inhibition attenuates plasma iron, oxidant stress, and renal injury following red blood cell transfusion in guinea pigs"; Transfusion 2020 Mar; 60(3):513-523*.*

The experiments described therein have been carried out by intravenously administering the small-molecule ferroportin inhibitor VIT-2653, corresponding to Example Compound No. 40 of the present invention and further confirm some of the findings of the present invention.

The NTBI and Hb levels following exchange transfusion were significantly improved by dosing of the ferroportin inhibitor.

Also, total iron in kidneys following transfusion can be reduced by dosing of the ferroportin inhibitor. The contribution of circulating Hb on renal iron loading and the subsequent effects on oxidative stress and cellular injury was evaluated revealing that dosing of the ferroportin inhibitor to transfused guinea pigs significantly reduced the occurrence of changes in plasma creatinine > 0.3 mg/dL, which is used as indicator of early acute kidney injury (AKI).

The experimental details and study conditions and the concrete study results can be derived from the mentioned paper.

## Claims

1. The compound according to the formula 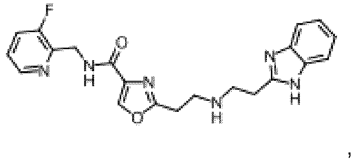 and pharmaceutically acceptable salts, solvates, hydrates and polymorphs thereof, for use in the prophylaxis or treatment of myelodysplastic syndromes (MDS) and/or the symptoms associated therewith in patients which are selected from individuals suffering from very-low-risk, low-risk or intermediate-risk of myelodysplastic syndromes according to the International Prognostic Scoring System (IPSS).

2. The compound as defined in claim 1, or its salts, solvates, hydrates and polymorphs, for the use according to claim 1, wherein the treatment of myelodysplastic syndromes (MDS) and/or the symptoms associated therewith comprises the treatment of ineffective hematopoiesis, in particular ineffective erythropoiesis.

3. The compound as defined in claim 1, or its salts, solvates, hydrates and polymorphs, for the use according to claim 1, wherein the treatment of myelodysplastic syndromes (MDS) and/or the symptoms associated therewith comprises amelioration, prevention or delay of leukemia evolution, reduction of bone marrow immature cells, reduction of myeloid expansion, reduction of the production of inflammatory cytokines as TNFα and IL-1β by macrophages, and/or improvement of the bone marrow microenvironment.

4. The compound as defined in claim 1, or its salts, solvates, hydrates and polymorphs, for the use according to any one of the preceding claims, wherein the patients to be treated are selected from individuals **characterized by** one or more of the following:
- suffering from myelodysplastic syndromes with ring sideroblasts according to World Health Organization criteria, **characterized by** either ≥15% ring sideroblasts, or ≥5% ring sideroblasts if an SF3B1 mutation is present, or with <5% bone marrow blasts;
- suffering from myelodysplastic syndromes having erythropoietin levels above 200 U per liter;
- suffering from erythroid dysplasia;
- suffering from cytopenia, in particular peripheral cytopenia;
- bone marrow blasts < 5%;
- peripheral blood blasts < 1%;
- suffering from myelodysplastic syndromes with reduced or lack of response to erythropoiesis-stimulating agents;
- suffering from myelodysplastic syndromes with chromosome 5q deletion (del[5q]);
- SF3B1-mutant patients;
- patients with significantly down-regulated PPOX and/or ABCB7 genes compared to healthy individuals;
- being transfusion-dependent or receiving regular red blood cell transfusions of ≥ 2 units per 8 weeks.

5. The compound as defined in claim 1, or its salts, solvates, hydrates and polymorphs, for the use according to any one of the preceding claims, wherein the patients are **characterized by**
a) showing detectable NTBI levels, and/or
b) having Hb levels below 8 g/dL, and/or
c) having an MCV between 50 and 70 10fL, and/or
d) having an MCH between 12 and 20 pg, and/or
e) having a TSAT level > 45%.

6. The compound as defined in claim 1, or its salts, solvates, hydrates and polymorphs, for the use according to any one of the preceding claims, wherein the patients to be treated are selected from transfusion-dependent patients, **characterized by** receiving regular blood transfusions, which includes
a) repeated blood transfusions of equal red blood cell (RBC) units in varying subsequent time intervals or
b) repeated blood transfusions of equal RBC units in equal subsequent time intervals or
c) repeated blood transfusions of varying RBC units in equal subsequent time intervals or
d) repeated blood transfusions of varying RBC units in varying subsequent time interval.

7. The compound as defined in claim 1, or its salts, solvates, hydrates and polymorphs, for the use according to any one of the preceding claims, wherein the treatment comprises the oral administration of the compound, its salts, solvates, hydrates or polymorphs, to a patient in need thereof.

8. The compound as defined in claim 1, or its salts, solvates, hydrates and polymorphs, for the use according to any one of the preceding claims, wherein the treatment comprises administering to a patient in need thereof a dose of 5 mg, 15 mg, 60 mg, 120 mg or 240 mg; preferably a dose of 120 mg for patients with > 50 kg body weight and of 60 mg for patients with < 50 kg body weight once or twice daily.

9. The compound as defined in claim 1 for the use according to any one of the preceding claims, wherein the compound is in the form of a pharmaceutically acceptable salt with acids from the group consisting of benzoic acid, citric acid, fumaric acid, hydrochloric acid,
lactic acid, malic acid, maleic acid, methanesulfonic acid, phosphoric acid, succinic acid, sulfuric acid, tartaric acid and toluenesulfonic acid,
preferably with acids from the group consisting of citric acid, hydrochloric acid, maleic acid, phosphoric acid and sulfuric acid;
and solvates, hydrates and polymorphs thereof.

10. The compound as defined in claim 1 for the use according to any one of the preceding claims, wherein the compound is in the form of a pharmaceutically acceptable salt selected from:
a 1:1 sulfate salt having the formula 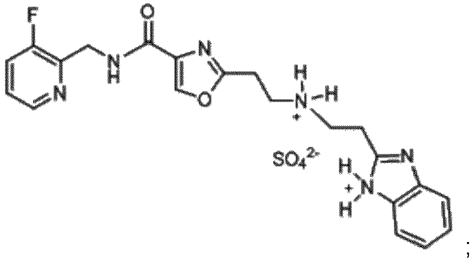
a 1:1 phosphate salt having the formula 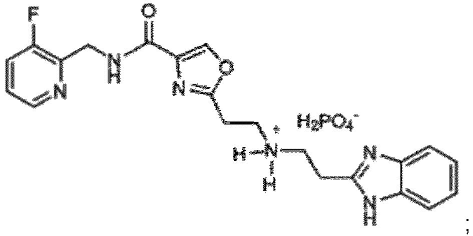
a 1:3 HCl salt having the formula 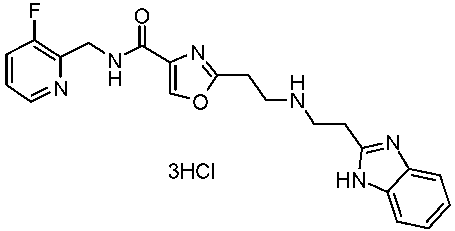
and polymorphs thereof.

11. A medicament containing one or more of the compounds as defined in any one of the claims 1, 9 and 10 for the use as defined in any one of the preceding claims, wherein the medicament further contains one or more pharmaceutical carriers and/or auxiliaries and/or solvents, and/or one or more additional pharmaceutically active compounds.

12. The medicament for use according to claim 11, comprising Luspatercept as an additional pharmaceutically active compound.

13. The compound as defined in claim 1, or its salts, solvates, hydrates and polymorphs, for the use in a combination therapy for treating myelodysplastic syndromes as defined in any one of the preceding claims, wherein the combination therapy comprises co-administration of the compound as defined in claim 1, including salts, solvates, hydrates and polymorphs thereof, with one or more other additional pharmaceutically active compounds,
wherein the co-administration of the combination therapy may be carried out in a fixed dose combination therapy by co-administration of the compound as defined in claim 1, including salts, solvates, hydrates and polymorphs thereof, with one or more other additional pharmaceutically active compounds in a fixed-dose formulation or
wherein the co-administration of the combination therapy may be carried out in a free dose combination therapy by co-administration of the compound as defined in claim 1, including salts, solvates, hydrates and polymorphs thereof, and the one or more other additional pharmaceutically active compounds in free doses of the respective compounds, either by simultaneous administration of the individual compounds or by sequential use of the individual compounds administered over a time period.

14. The compound for the use according to claim 13, wherein the additional pharmaceutically active compound is Luspatercept.

## Patentansprüche

1. Die Verbindung gemäß der Formel 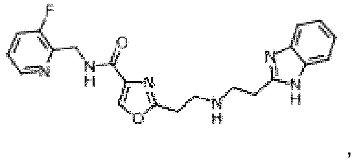 sowie deren pharmazeutisch verträgliche Salze, Solvate, Hydrate und Polymorphe zur Verwendung bei der Prophylaxe oder Behandlung von myelodysplastischen Syndromen (MDS) und/oder den damit verbundenen Symptomen bei Patienten, die aus Personen ausgewählt sind, die gemäß dem International Prognostic Scoring System (IPSS) an myelodysplastischen Syndromen mit sehr geringem, geringem oder mittlerem Risiko leiden.

2. Die in Anspruch 1 definierte Verbindung oder deren Salze, Solvate, Hydrate und Polymorphe zur Verwendung gemäß Anspruch 1, wobei die Behandlung von myelodysplastischen Syndromen (MDS) und/oder der damit verbundenen Symptome die Behandlung einer ineffektiven Hämatopoese, insbesondere einer ineffektiven Erythropoese, umfasst.

3. Die in Anspruch 1 definierte Verbindung oder deren Salze, Solvate, Hydrate und Polymorphe zur Verwendung gemäß Anspruch 1, wobei die Behandlung von myelodysplastischen Syndromen (MDS) und/oder der damit verbundenen Symptome die Linderung, Vorbeugung oder Verzögerung des Leukämieverlaufs, die Verringerung unreifer Knochenmarkszellen, die Verringerung der myeloischen Expansion, die Verringerung der Produktion von entzündlichen Zytokinen wie TNFα und IL-1β durch Makrophagen und/oder die Verbesserung der Knochenmark-Mikroumgebung umfasst.

4. Die in Anspruch 1 definierte Verbindung oder deren Salze, Solvate, Hydrate und Polymorphe zur Verwendung gemäß einem der vorstehenden Ansprüche, wobei die zu behandelnden Patienten aus Personen ausgewählt sind, die durch eines oder mehrere der folgenden Merkmale gekennzeichnet sind:
- die an myelodysplastischen Syndromen mit Ringsideroblasten gemäß den Kriterien der Weltgesundheitsorganisation leiden, **gekennzeichnet durch** entweder ≥ 15 % Ringsideroblasten oder ≥ 5 % Ringsideroblasten, falls eine SF3B1-Mutation vorliegt, oder mit < 5 % Knochenmarkblasten;
- die an myelodysplastischen Syndromen mit Erythropoietin-Spiegeln über 200 U pro Liter leiden;
- die an einer erythroiden Dysplasie leiden;
- die an einer Zytopenie, insbesondere einer peripheren Zytopenie, leiden;
- Knochenmarkblasten < 5 %;
- periphere Blutblasten < 1 %;
- die an myelodysplastischen Syndromen leiden und auf erythropoetische Wirkstoffe nur unzureichend oder gar nicht ansprechen;
- die an myelodysplastischen Syndromen mit einer Deletion des Chromosoms 5q (del[5q]) leiden;
- Patienten mit SF3B1-Mutation;
- Patienten mit signifikant herunterregulierten PPOX- und/oder ABCB7-Genen im Vergleich zu gesunden Personen;
- die transfusionsabhängig sind oder regelmäßig Erythrozytentransfusionen von ≥ 2 Einheiten pro 8 Wochen erhalten.

5. Die in Anspruch 1 definierte Verbindung oder deren Salze, Solvate, Hydrate und Polymorphe zur Verwendung gemäß einem der vorstehenden Ansprüche, wobei die Patienten **dadurch gekennzeichnet sind, dass** sie
a) nachweisbare NTBI-Spiegel und/oder
b) Hb-Spiegeln unter 8 g/dL, und/oder
c) einen MCV-Wert zwischen 50 und 70 10fL und/oder
d) einen MCH-Wert zwischen 12 und 20 pg und/oder
e) einen TSAT-Wert von > 45 %
aufweisen.

6. Die in Anspruch 1 definierte Verbindung oder deren Salze, Solvate, Hydrate und Polymorphe zur Verwendung gemäß einem der vorstehenden Ansprüche, wobei die zu behandelnden Patienten aus transfusionsabhängigen Patienten ausgewählt sind, die sich dadurch charakterisieren, dass sie regelmäßige Bluttransfusionen erhalten, welche beinhalten
a) wiederholte Bluttransfusionen mit gleichen Einheiten roter Blutkörperchen (RBC) in unterschiedlichen nachfolgenden Zeitintervallen oder
b) wiederholte Bluttransfusionen mit gleichen RBC-Einheiten in gleichen Zeitintervallen oder
c) wiederholte Bluttransfusionen mit unterschiedlichen RBC-Einheiten in gleichen Zeitintervallen oder
d) wiederholte Bluttransfusionen mit unterschiedlichen RBC-Einheiten in unterschiedlichen nachfolgenden Zeitintervallen.

7. Die in Anspruch 1 definierte Verbindung oder deren Salze, Solvate, Hydrate und Polymorphe zur Verwendung gemäß einem der vorstehenden Ansprüche, wobei die Behandlung die orale Verabreichung der Verbindung, ihrer Salze, Solvate, Hydrate oder Polymorphe an einen Patienten umfasst, der diese benötigt.

8. Die in Anspruch 1 definierte Verbindung oder deren Salze, Solvate, Hydrate und Polymorphe zur Verwendung gemäß einem der vorstehenden Ansprüche, wobei die Behandlung die Verabreichung einer Dosis von 5 mg, 15 mg, 60 mg, 120 mg oder 240 mg an einen Patienten umfasst, der diese benötigt; vorzugsweise eine Dosis von 120 mg für Patienten mit einem Körpergewicht von > 50 kg und von 60 mg für Patienten mit einem Körpergewicht von < 50 kg, ein- oder zweimal täglich.

9. Die in Anspruch 1 definierte Verbindung zur Verwendung gemäß einem der vorstehenden Ansprüche, wobei die Verbindung in Form eines pharmazeutisch verträglichen Salzes mit Säuren aus der Gruppe bestehend aus Benzoesäure, Zitronensäure, Fumarsäure, Salzsäure, Milchsäure, Apfelsäure, Maleinsäure, Methansulfonsäure, Phosphorsäure, Bernsteinsäure, Schwefelsäure, Weinsäure und Toluolsulfonsäure,
vorzugsweise mit Säuren aus der Gruppe bestehend aus Zitronensäure, Salzsäure, Maleinsäure, Phosphorsäure und Schwefelsäure;
sowie deren Solvate, Hydrate und Polymorphe.

10. Die in Anspruch 1 definierte Verbindung zur Verwendung gemäß einem der vorstehenden Ansprüche, wobei die Verbindung in Form eines pharmazeutisch verträglichen Salzes vorliegt, ausgewählt aus:
einem 1:1-Sulfatsalz der Formel 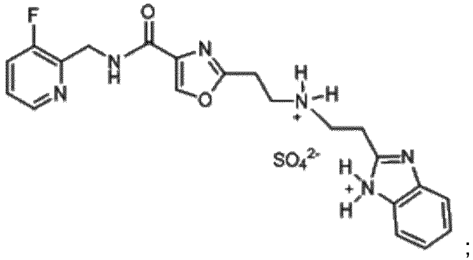
einem 1:1-Phosphatsalz der Formel 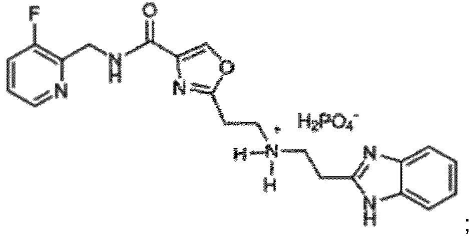
einem 1:3-HCl-Salz der Formel 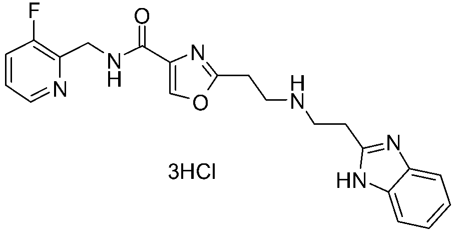
sowie deren Polymorphe.

11. Arzneimittel, das eine oder mehrere der in einem der Ansprüche 1, 9 und 10 definierten Verbindungen zur in einem der vorstehenden Ansprüche definierten Verwendung enthält, wobei das Arzneimittel ferner einen oder mehrere pharmazeutische Trägerstoffe und/oder Hilfsstoffe und/oder Lösungsmittel und/oder eine oder mehrere zusätzliche pharmazeutisch wirksame Verbindungen enthält.

12. Das Arzneimittel zur Verwendung gemäß Anspruch 11, umfassend Luspatercept als zusätzliche pharmazeutisch wirksame Verbindung.

13. Die in Anspruch 1 definierte Verbindung oder deren Salze, Solvate, Hydrate und Polymorphe zur Verwendung in einer Kombinationstherapie zur Behandlung von myelodysplastischen Syndromen, wie in einem der vorstehenden Ansprüche definiert, wobei die Kombinationstherapie die gleichzeitige Verabreichung der in Anspruch 1 definierten Verbindung, einschließlich deren Salze, Solvate, Hydrate und Polymorphe, zusammen mit einer oder mehreren weiteren pharmazeutisch wirksamen Verbindungen umfasst,
wobei die gleichzeitige Verabreichung der Kombinationstherapie in Form einer Festdosis-Kombinationstherapie durch die gleichzeitige Verabreichung der in Anspruch 1 definierten Verbindung, einschließlich ihrer Salze, Solvate, Hydrate und Polymorphe, zusammen mit einer oder mehreren weiteren pharmazeutisch wirksamen Verbindungen in einer Festdosis-Formulierung erfolgen kann oder
wobei die gemeinsame Verabreichung der Kombinationstherapie in einer Kombinationstherapie mit festen Dosierungen durch gemeinsame Verabreichung der in Anspruch 1 definierten Verbindung, einschließlich ihrer Salze, Solvate, Hydrate und Polymorphe, und der einen oder mehreren weiteren pharmazeutisch wirksamen Verbindungen in freien Dosierungen der jeweiligen Verbindungen erfolgen kann, entweder durch gleichzeitige Verabreichung der einzelnen Verbindungen oder durch aufeinanderfolgende Anwendung der einzelnen Verbindungen über einen Zeitraum hinweg.

14. Die Verbindung zur Verwendung gemäß Anspruch 13, wobei die zusätzliche pharmazeutisch wirksame Verbindung Luspatercept ist.

## Revendications

1. Composé répondant à la formule 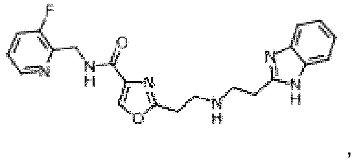 ainsi que ses sels, solvates, hydrates et polymorphes pharmaceutiquement acceptables, destinés à être utilisés dans la prophylaxie ou le traitement des syndromes myélodysplasiques (SMD) et/ou des symptômes qui y sont associés chez des patients choisis parmi les personnes présentant un risque très faible, faible ou intermédiaire de syndromes myélodysplasiques selon le système international de score pronostique (IPSS).

2. Le composé tel que défini dans la revendication 1, ou ses sels, solvates, hydrates et polymorphes, destiné à l'utilisation selon la revendication 1, dans lequel le traitement des syndromes myélodysplasiques (SMD) et/ou des symptômes qui y sont associés comprend le traitement d'une hématopoïèse inefficace, en particulier d'une érythropoïèse inefficace.

3. Le composé tel que défini dans la revendication 1, ou ses sels, solvates, hydrates et polymorphes, destiné à l'utilisation selon la revendication 1, dans laquelle le traitement des syndromes myélodysplasiques (SMD) et/ou des symptômes qui y sont associés comprend l'atténuation, la prévention ou le retardement de l'évolution de la leucémie, la réduction des cellules immatures de la moelle osseuse, la réduction de l'expansion myéloïde, la réduction de la production de cytokines inflammatoires telles que TNFα et IL-1β par les macrophages, et/ou l'amélioration du microenvironnement de la moelle osseuse.

4. Le composé tel que défini dans la revendication 1, ou ses sels, solvates, hydrates et polymorphes, destiné à l'utilisation selon l'une quelconque des revendications précédentes, dans lequel les patients à traiter sont choisis parmi des individus présentant l'une ou plusieurs des caractéristiques suivantes :
- souffrant de syndromes myélodysplasiques avec sidéroblastes en anneau selon les critères de l'Organisation mondiale de la santé, caractérisés soit par ≥ 15 % de sidéroblastes en anneau, soit par ≥ 5 % de sidéroblastes en anneau en cas de présence d'une mutation du gène SF3B1, soit par < 5 % de blastes médullaires ;
- souffrant de syndromes myélodysplasiques présentant des taux d'érythropoïétine supérieurs à 200 U par litre ;
- souffrant d'une dysplasie érythroïde ;
- souffrant de cytopénie, en particulier de cytopénie périphérique ;
- présentant un taux de blastes médullaires inférieur à 5 % ;
- pourcentage de blastes dans le sang périphérique inférieur à 1 % ;
- atteints de syndromes myélodysplasiques présentant une réponse réduite ou absente aux agents stimulant l'érythropoïèse ;
- atteints de syndromes myélodysplasiques avec délétion du chromosome 5q (del[5q]) ;
- les patients présentant une mutation du gène SF3B1 ;
- patients présentant une régulation à la baisse significative des gènes PPOX et/ou ABCB7 par rapport à des individus sains ;
- dépendants des transfusions ou recevant régulièrement des transfusions de globules rouges à raison d'au moins 2 unités toutes les 8 semaines.

5. Le composé tel que défini dans la revendication 1, ou ses sels, solvates, hydrates et polymorphes, destiné à l'utilisation selon l'une quelconque des revendications précédentes, dans lequel les patients sont **caractérisés par**
a) des taux détectables de NTBI, et/ou
b) des taux d'hémoglobine inférieurs à 8 g/dL, et/ou
c) un VGM compris entre 50 et 70 10fL, et/ou
d) un MCH compris entre 12 et 20 pg, et/ou
e) un taux de TSAT > 45 %.

6. Le composé tel que défini dans la revendication 1, ou ses sels, solvates, hydrates et polymorphes, destiné à l'utilisation selon l'une quelconque des revendications précédentes, dans lequel les patients à traiter sont choisis parmi les patients dépendants des transfusions, **caractérisés par** le fait de recevoir des transfusions sanguines régulières, ce qui inclut
a) des transfusions sanguines répétées d'unités égales de globules rouges (RBC) à des intervalles de temps successifs variables ou
b) des transfusions sanguines répétées d'unités de RBC identiques à des intervalles de temps successifs identiques, ou
c) des transfusions sanguines répétées d'unités de RBC variables à des intervalles de temps successifs égaux, ou
d) des transfusions sanguines répétées d'unités de RBC variables à des intervalles de temps successifs variables.

7. Le composé tel que défini dans la revendication 1, ou ses sels, solvates, hydrates et polymorphes, destiné à l'utilisation selon l'une quelconque des revendications précédentes, dans lequel le traitement comprend l'administration par voie orale du composé, de ses sels, solvates, hydrates ou polymorphes, à un patient qui en a besoin.

8. Le composé tel que défini dans la revendication 1, ou ses sels, solvates, hydrates et polymorphes, destiné à l'utilisation selon l'une quelconque des revendications précédentes, dans lequel le traitement consiste à administrer à un patient qui en a besoin une dose de 5 mg, 15 mg, 60 mg, 120 mg ou 240 mg ; de préférence une dose de 120 mg pour les patients dont le poids corporel est supérieur à 50 kg et de 60 mg pour les patients dont le poids corporel est inférieur à 50 kg, une ou deux fois par jour.

9. Le composé tel que défini dans la revendication 1, destiné à l'utilisation selon l'une quelconque des revendications précédentes, **caractérisé en ce que** le composé se présente sous la forme d'un sel pharmaceutiquement acceptable avec des acides choisis dans le groupe constitué par l'acide benzoïque, l'acide citrique, l'acide fumarique, l'acide chlorhydrique, l'acide lactique, l'acide malique, l'acide maléique, l'acide méthanesulfonique, l'acide phosphorique, l'acide succinique, l'acide sulfurique, l'acide tartrique et l'acide toluènesulfonique,
de préférence avec des acides choisis parmi le groupe comprenant l'acide citrique, l'acide chlorhydrique, l'acide maléique, l'acide phosphorique et l'acide sulfurique ;
ainsi que leurs solvates, hydrates et polymorphes.

10. Le composé tel que défini dans la revendication 1, destiné à l'utilisation selon l'une quelconque des revendications précédentes, dans lequel le composé se présente sous la forme d'un sel pharmaceutiquement acceptable choisi parmi :
un sel de sulfate 1:1 répondant à la formule 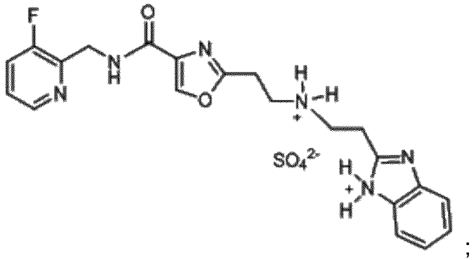
un sel de phosphate 1:1 répondant à la formule 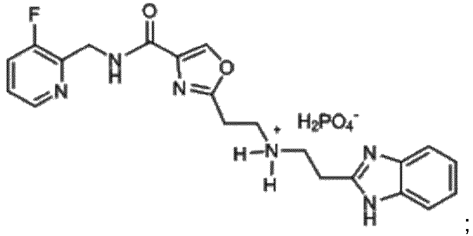
un sel de HCl 1:3 répondant à la formule 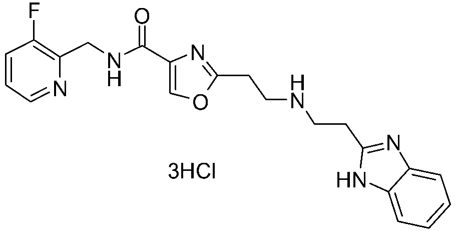 et ses polymorphes.

11. Médicament contenant un ou plusieurs des composés tels que définis dans l'une quelconque des revendications 1, 9 et 10, destiné à l'utilisation telle que définie dans l'une quelconque des revendications précédentes, dans lequel le médicament contient en outre un ou plusieurs excipients et/ou adjuvants et/ou solvants pharmaceutiques, et/ou un ou plusieurs composés pharmaceutiquement actifs supplémentaires.

12. Le médicament destiné à l'utilisation selon la revendication 11, comprenant du luspatercept en tant que composé pharmaceutiquement actif supplémentaire.

13. Le composé tel que défini dans la revendication 1, ou ses sels, solvates, hydrates et polymorphes, destiné à être utilisé dans un traitement combiné pour le traitement des syndromes myélodysplasiques tels que définis dans l'une quelconque des revendications précédentes, dans lequel le traitement combiné comprend l'administration conjointe du composé tel que défini dans la revendication 1, y compris ses sels, solvates, hydrates et polymorphes, avec un ou plusieurs autres composés pharmaceutiquement actifs supplémentaires,
dans laquelle la co-administration du traitement combiné peut être réalisée dans le cadre d'un traitement combiné à dose fixe par co-administration du composé tel que défini dans la revendication 1, y compris ses sels, solvates, hydrates et polymorphes, avec un ou plusieurs autres composés pharmaceutiquement actifs supplémentaires dans une formulation à dose fixe, ou
dans laquelle l'administration conjointe du traitement combiné peut être réalisée dans le cadre d'un traitement combiné à doses libres par l'administration conjointe du composé tel que défini dans la revendication 1, y compris ses sels, solvates, hydrates et polymorphes, et d'un ou plusieurs autres composés pharmaceutiquement actifs supplémentaires à des doses libres des composés respectifs, soit par administration simultanée des composés individuels, soit par utilisation séquentielle des composés individuels administrés sur une période donnée.

14. Composé destiné à l'utilisation selon la revendication 13, dans lequel le composé pharmaceutiquement actif supplémentaire est le luspatercept.
